# EUROPEAN PATENT APPLICATION

(11) **EP 2 626 363 A1**
(43) Date of publication of application: **14.08.2013**
(21) Application number: 11821149.9
(22) Date of filing: 25.08.2011
(51) Int. Cl.: C07K 1/14, C07K 1/36

(54) **METHOD FOR PRODUCING AND PURIFYING RECOMBINANT PROTEINS IN PLANTS**

(30) Priority: 31.08.2010 ES 201001115
(71) Applicant: Idén Biotechnology, S.L., 31110 Noain Navarra (ES)
(72) Inventor: BAROJA FERNANDEZ, Miren Edurne, E-31192 Mutilva (NAVARRA) (ES); POZUETA ROMERO, Javier, E-31192 Mutilva (NAVARRA) (ES); MUÑOZ PEREZ, Francisco josé, E-31192 Mutilva (NAVARRA) (ES); ABDELLATIF, Bahaji, E-31192 Mutilva (NAVARRA) (ES)
(74) Representative: Elzaburu Marquez, Alberto
(86) International application number: PCT/ES2011/000266
(87) International publication number: WO 2012/028753

(57) **Abstract**

The invention relates to a method for producing and purifying recombinant proteins in plants, by means of producing transgenic plants expressing the recombinant proteins fused with starch granule-associated proteins through a sequence recognized by a protease. This method allows purifying the protein by means of simple steps of homogenizing, centrifuging and treating with protease. Likewise, the invention relates to the vectors, hosts, cells and plants used in said method.

## Description

### Field of the Invention

The present invention is encompassed within the field of the genetic engineering and biotechnology. Specifically, the invention relates to a method for obtaining purified heterologous recombinant proteins by means of producing transgenic plants expressing the recombinant proteins fused with starch granule-associated proteins through a sequence recognized by a protease. This method allows purifying the protein by means of simple steps of homogenizing, centrifuging and treating with protease. Likewise, the invention relates to the vectors, hosts, cells and plants used in said method.

### Background of the Invention

The use of plants as biofactories (molecular farming) relates to the use of plants to produce high added value compounds that can be applied to different industries such as the pharmaceutical industry, food industry, chemical industry, etc. It aims to genetically modify plants for the purpose of producing the compound of interest.

The systems for producing recombinant proteins of industrial interest currently use bacteria, yeasts and mammal cell culture, but each of these systems has its limitations. The use of plants as biofactories has economical advantage (lower costs in the large scale production), product safety advantage (the risk of introducing pathogens from animal tissues in the extraction and purification processes is prevented) and technical advantage (in some cases the plants can form the only system capable of efficiently producing certain human proteins such as growth regulators and cell cycle inhibitors, which would have a negative impact in the transgenic animals or in the cultured animal cells in which they will be expressed) (Giddings 2001; Ma *et al.* 2003; Twyman et al. 2003). On the other hand, proteins expressed in seeds can remain stable for years at room temperature without losing their activity (Giddings 2001; Ma *et al.* 2003). Currently proteins of therapeutic interest are being produced in a wide variety of plants including tobacco, cereals, oil plants, fruits, vegetables, forage crops and single-cell algae (Twyman *et al.* 2003; Fischer *et al.* 2004).

The recombinant proteins expressed in bacteria are proteins which do not require glycosylation to be active and are generally accumulated in insoluble aggregates the purification and renaturation costs of which are not sustainable. When the recombinant proteins require post-translational modification processes other expression systems such as mammal cell cultures involving a high starting cost for obtaining them and large additional investments when it is required to increase the production scale, are used. This problem can be solved by means of using plants as biofactories, since the protein synthesis and secretion mechanisms and the post-translational modifications are typical of the eukaryotic cells and allow the production and assembly of complex proteins.

The current objectives of the "molecular farming" industry are directed to obtaining new products and increasing the productivity. The latter is largely determined by the production and purification processes (Twyman *et al.* 2005).

Most of the production costs of a recombinant protein are not incurred in the production phase, but in the subsequent purification processes of the product. Such as indicated in the recent Ward and Swiatek review (Ward and Swiatek, 2009), obtaining the pure protein is not simple. Even in the case of the recombinant proteins produced in an expression system for which the production method are previously designed and optimized, relatively few proteins can be purified to a purity of 99% by means of methods having a single step, commonly one or more additional steps being needed. The main part of the purification protocols starts with fast low resolution steps allowing a high degree of the protein of interest recovery and the elimination of turbidity such as the differential precipitation with ammonium sulfate followed by centrifugation. The fine purification of the protein is commonly performed by chromatography methods, the affinity chromatography for the recombinant proteins being fairly popular. It is, however, an expensive methodology requiring several stages until obtaining the purified protein. Other non-chromatography techniques such as electrophoresis have practical use as analytic instead of preparative tools which facilitate estimating the purity of a protein, but do not allow separating amounts greater than micrograms.

In addition to the cost of the purification methods and the number of steps which can be necessary to obtain a protein with the required degree of purity, another additional problem may be the integrity of the protein. This problem is especially significant when the protein is to be obtained for pharmaceutical applications, wherein the protein is necessarily integral and is also in a stable conformation, in which the biological activity thereof takes place. The significance of this problem is clearly seen, for example, when concentrating the recombinant protein production in certain organs of the plants must be attempted or upon resorting to expression technologies locating the recombinant proteins in an organelle particularly of the cells which facilitates the purification thereof. To that end, it is common to resort to designing of those so-called "fusion proteins", polypeptide chains consisting of two or more polypeptides bound in a single protein. Each of the polypeptides commonly comes from a different origin and in any case, the polypeptides making up the hybrid polypeptide or fusion protein are not naturally found forming a single polypeptide. To produce fusion proteins, the corresponding coding sequences are inserted into vectors whereby transgenic microorganisms, plants or animals are transformed, in which the fusion proteins are synthesized as if they were the typical proteins. Subsequently, the proteins are purified from the corresponding transgenic microorganism or plant or animal tissue sample cultures.

The concentration of fusion protein production in plant organs or specific organelles of their cells had not solved the problem of the difficulty for purification and the need of resorting to several usually expensive steps for obtaining the required degree of purification. This has been, for example, with the fusion of proteins of interest to the oleosin, a type of membrane protein forming part of the oil bodies which are organelles existing in the cytosol of some seeds specialized in accumulating oil (Markley *et al.* 2006). The method for purifying oleosin-bound recombinant proteins is based on homogenizing tissues and then purifying the oil bodies of transgenic plants after a single pass of homogenate centrifugation and floating the oil bodies. This, however, does not prevent many soluble proteins from contaminating the oil body preparations, making the subsequent purification of oleosin-bound recombinant proteins difficult. Furthermore, oleosins have the specific problem of having allergenic properties, therefore they must be completely removed during the purification process, especially if the protein to be purified is to be used for its administration into human beings; all this further complicates the purification process. The synthesis of proteins fused to oleosins is also not a method with a high relative yield since the oleosins are only located on the surface of the oil bodies, not inside them, therefore the total amount of fusion protein obtained with respect to total weight of the oil body is not high.

Other similar techniques used are the use of small hydrophobic proteins (hydrophobins) fused to the recombinant protein facilitating its purification (Joensuu *et al.* 2010), the production of recombinant proteins which contain integration sequences in the cell plasma membrane (Schillberg *et al.* 2000) or the secretion of the recombinant proteins to the extracellular space or apoplast (Borisjuk *et al.* 1999). However, all these techniques for purifying recombinant proteins have similar problems as those previously described based on the use of recombinant oleosins, especially in that relating to the difficulty for obtaining the desired protein with a high degree of purity.

The international patent application WO 98/14601 describes fusion proteins in which the polypeptide of interest is bound to what the applicant called "encapsulating region in the starch" (SER: starch-encapsulating region). This region allows associating the starch granules with enzymes involved in their metabolism such as the soluble starch synthases I, II or III (SSI, SSII or SSIII), granule-bound starch synthase (GBSS), branching enzymes I, IIa and IIBb or glucoamylase. Thus, when the fusion protein is expressed in a plant, it is encapsulated in the starch granule. The main proposal of the application is obtaining modified starch granules which can be used directly such as to fortify animal feeds with certain amino acids or to provide hormones or drugs of interest together with the food, which will be protected from degradation in the stomach by the starch itself to which they are bound. Therefore, the purification of the protein of interest prior to its administration to an animal is prevented. The application also suggests the possibility of purifying the fusion protein by means of affinity columns in the manner similar to that mentioned in previous patents, such as the USA patent US 5202247, related to hybrid proteins capable of binding to a polysaccharide matrix. Document WO 98/14601 also suggests the possible presence of an excision sequence between the starch-encapsulating region and the polypeptide of interest facilitating the separation thereof from the remaining portion of the fusion protein and the subsequent independent purification. In addition to the excision by means of proteolysis, the excision is performed by means of chemical methods such as decreasing the pH or using cyanobromide. Another chromatography method, the ion exchange chromatography, is proposed as an alternative method for purifying the desired polypeptide when polyarginine tails have been added to the recombinant fusion protein.

The description of WO 98/14601 even provides a diagram of the structure that the DNA construct must have to express the hybrid polypeptide in the host, in which the order of the elements is the following: promoter - intron (optional) -coding region of the transit peptide - coding region of the polypeptide of interest - SER- terminator. The inclusion of the transit peptide is correctly justified so that the protein is translocated to organelles such as the plastids, showing preference for the amyloplasts. However, as will be discussed below, this structure entails problems when purifying the polypeptide of interest bound to the starch granule, which problems are also shown if the international application WO 00/77165 is analyzed.

International application WO 00/77165 does relate specifically to the expression of fusion proteins in plants, in which the fusion protein has SBD (starch-binding domain). In said international application SBD is considered both as the starch-encapsulating region which is referred to in the international application WO 98/14601 and the starch-binding domains having some amylolytic enzymes expressed by microorganisms. However, it is preferably expressed in that the protein of interest bound to the SBD is an enzyme which can interact with the starch such that starch modified in its properties not only by the simple presence of the exogenous protein but by the activity thereof, is obtained. Nor does it require that the protein bound to the SBD is complete, although preference is seen in that the chosen fragment gave rise to fusion protein which maintains the biological activity of the protein of interest. Even so, the possibility of using the method to produce proteins in commercial amounts, isolating them with the starch granule or separating them from the fusion protein, taking advantage of the presence of an excision site, is suggested. For the purification in the form of fusion protein, it is mentioned that the use of the SBDs offers the possibility of using them as affinity tails whereby they facilitate the purification of the proteins bound to such SBD by means of affinity chromatography through starch columns which seems to be the preferred purification method since the same is referred to in several occasions. Therefore, it can be considered that the protein purification system suggested in application WO 00/77165, similarly that in WO 98/14601, would consist of: (a) producing recombinant proteins bound to a "starch binding domain" (SBD) in bacteria, vegetable or animal cells, (b) extracting by means of rupturing/homogenizing/grinding the cells/tissue/organ and (c) purifying the protein by means of affinity chromatography in a starch column to which the recombinant proteins having a SBD will be specifically bound. The subsequent release of the protein from the starch can take place by means of adding an endoprotease which specifically recognizes an amino acid sequence existing between the SBD and the protein of interest. The purification of the recombinant protein depends on the use of a very well-known system at the time when WO 98/14601 was filed, the affinity chromatography through starch columns.

Most of the chloroplast proteins are synthesized in the form of precursors in the cytosol. These precursors contain cleavable signals in the amino end known as transit peptides which direct them to the chloroplasts. The precursor proteins are translocated through the chloroplast membrane by means of the combined action of the Toc translocon (in the outer membrane) and Tic translocon (in the inner membrane of the chloroplasts), forming the toc-tic system (Jarvis and Soll, 2002). The toc-tic system for processing plastidial proteins is not entirely accurate. It is based on recognizing non-predictable hydrophobic domains which perform the cleavage to split the transit peptide in an exact point. Thus, the programs for predicting the sub-cellular localization and the processing site of a protein bound to a transit peptide tend to have many problems when performing (a) the exact sub-cellular localization prognosis of the protein and (b) the cleavage/processing site prognosis of the transit peptide (Bahaji *et al.* 2011). It is very difficult to find a sequence acting as a plastid transit peptide which, after being removed, allows the protein incorporated to the amyloplast completely and exactly enters with the sequence which is desired in the N-terminal end thereof. Furthermore, there isn't a "model" amino acid sequence which, fused to any protein, assures the transportation of the protein to the plastid integrally.

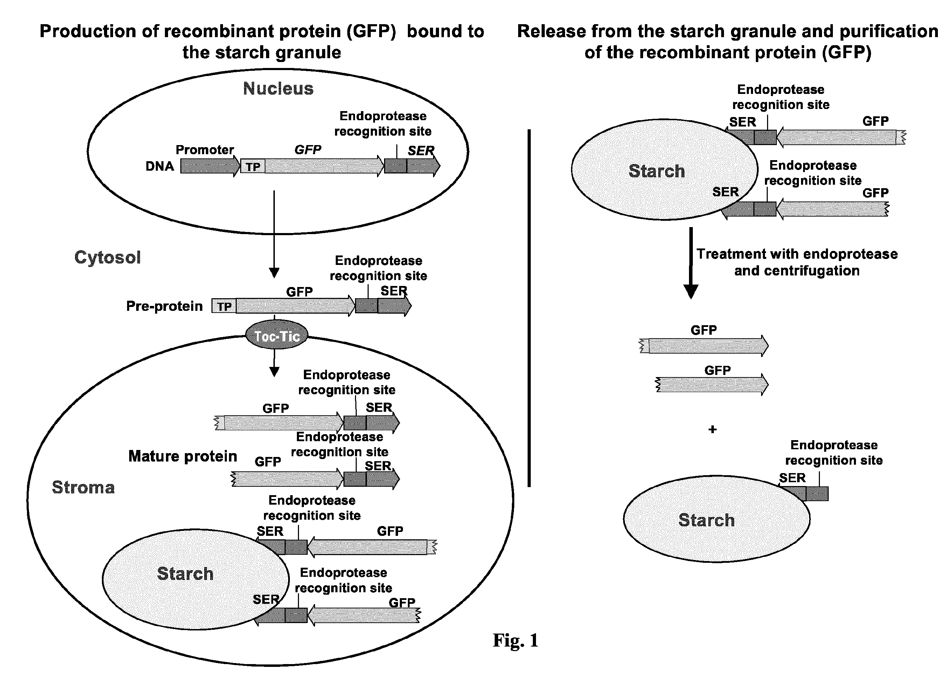

Therefore, it is highly probable that, after being processed, the protein hybridized by the toc-tic system existing in the plastid membrane, the protein of interest incorporated has at its N-terminal end a "residue" or vestige of the C-terminal end of the transit peptide. This involves a problem for the practical application of the methods of applications WO98/14601 and WO 00/77165 when they are to be used for purifying the protein of interest if, as indicated in said applications, the starch binding domain is placed in the carboxyl end of the fusion protein. As depicted in Figure 1, when the fusion protein penetrates the plastid with the starch binding domain (represented in this case by a SER, a starch-encapsulating domain), it is highly probable that the transit peptide has been split such that the polypeptide of interest (represented in this case by the GFP) has part of the amino acids of the transit peptide or lacks some of the amino acids of its amino end, a defect which will remain once the fusion protein is purified and the final excision is made, separating it from the part which contain the starch binding domain. If the protein is to be used in the pharmaceutical sector, (where, as has been explained previously, modifications in the amino acid sequences of therapeutic peptides are not allowed), this would be unacceptable and the protein would be rejected for use in that context.

This is not the only problem of the application WO 98/14601. In addition to not having any specific example of fusion proteins production in plants and bacteria and the subsequent purification of the protein of interest, the description has other passages putting in doubt the usefulness, in which a person skilled in the art would grant their teachings as a reliable information that enables a skilled person to develop and carry out a method for purifying proteins from starch-associated fusion polypeptides expressed in plants based on the content of said application. Said application WO 98/14601 mentions that the hybrid polypeptide (produced in plants or bacteria) can include post-translational modifications known in the technique such as glycosylation, acylation, and other modifications which are necessary for the polypeptide activity. This statement hardly goes with the proposal/idea of producing plants intended for purifying recombinant proteins after releasing them from the "transgenic starch" granule since the glycosylation (particularly the N-glycosylation) takes place in endoplasmic reticulum system/Golgi system. The glycosylated proteins are secreted or remain in the membranes (plasma membrane, tonoplast, etc) or organelles of the secretory pathway. At the time of the application of WO 98/14601 the existence of glycosylated plastidial proteins was not known. And, although much later on, Villarejo *et al.* (2005), Nanjo et *al.* (2006) and Kitajima *et al.* (2009) demonstrated the existence of a N-glycosylated protein secretory pathway between the ER/Golgi and the plastidial compartment, up until now producing transgenic plants which accumulate glycosylated proteins of interest in their plastids has not been achieved. Furthermore, this statement is incompatible with the proposal of producing glycosylated proteins in bacteria since, as it is well known, bacteria do not glycosylate proteins.

In conclusion, there is a need of finding alternative methods for purifying proteins forming part of fusion proteins which are simple to perform, which require a reduced number of steps to achieve a protein with a high degree of purity (especially in the case of the proteins which are used in the pharmaceutical sector) and which are, preferably, more economical than the chromatography methods, especially affinity chromatography, which are mainly resorted to at present. The expression of the corresponding fusion proteins associated to specific organelles had not solved this problem and in some cases, such as in that of the association to oleosins, it has additional problems such as the allergenic potential. The related methodologies in which fusion proteins have been directed to the starch granule also do not have actual and effective purification alternatives substituting the affinity columns and, furthermore, some problems are left pending without being solved for the application in specific fields, it can be, such as the design of the systems which assures obtaining the protein of interest integrally, enabling the use in the pharmaceutical sector. The resolution of these problems is important to consider "molecular farming" as a profitable business.

The present invention presents a solution to these problems.

### Brief Description of the Invention

The present invention solves the problem for the large scale purification of recombinant proteins by means of a simple and low cost system, suitable to be used for purifying proteins of interest from fusion polypeptides expressed in plants and, if necessary, making obtaining the integral protein without loosing additional fragments or amino acids in any end possible.

The proposed solution consists of a method based on purifying the fusion proteins from transgenic plants which ectopically express the recombinant protein (the polypeptide of industrial/commercial interest) fused with a polypeptide fragment having a starch binding domain. The fusion protein has several significant particularities:
a) the polypeptide fragment having the starch binding domain is bound to the protein of interest by means of a fragment having a protease cleavage sequence which facilitates their separation;
b) the protein of interest is in the carboxyl end region of the fusion protein, the polypeptide fragment having the starch binding domain being in the amino end region; the fragment having the protease cleavage sequence therefore links the carboxyl end of the polypeptide fragment having the starch binding domain with the amino end of the polypeptide of interest;
c) the polypeptide fragment having the starch binding domain can comprise:
   i) either a plant protein (e.g. GBSS) chosen from the group which is known to associate to the starch granule (protein which will not, therefore, only have a starch binding domain naturally but, it will also have, in its synthesized precursor form in the cytosol, a typical transit peptide which will direct it to the plastid where it will be associated to the starch), or
   ii) a bacterial amylase starch binding domain which will be preceded by a transit peptide of a plant protein which is associated to the starch granule which will allow the synthesized form in the cytosol to reach the plastid where it will be associated to the starch.

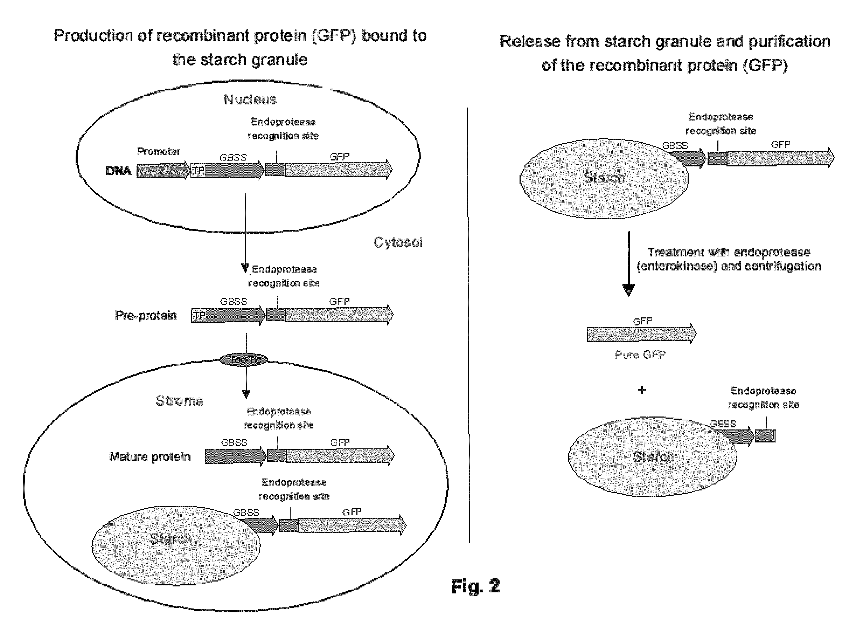

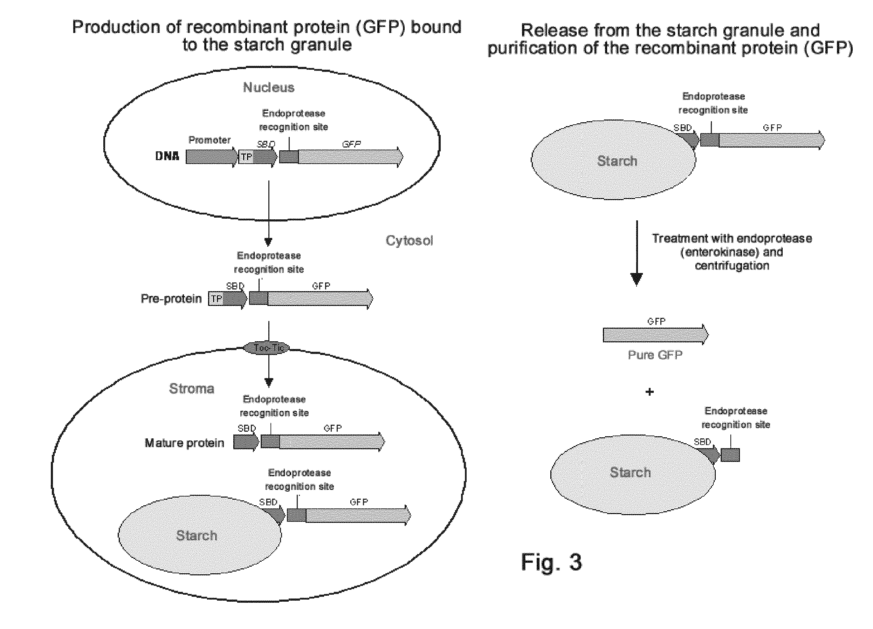

With this strategy, the purification method from transgenic plants ectopically expressing proteins fused with proteins bound to the starch granule (e.g.: GBSS) through a sequence recognized by a protease. Thus, the protein of interest, together with the rest of the fusion protein, will be anchored to the starch granule. The high density of the starch granules allows an easy purification of the rest of cellular components after a single pass of tissue homogenization and centrifugation. The subsequent treatment with protease allows releasing the protein of interest which can be obtained in a pure form simply after a new centrifugation and protease removal. Therefore, the new method proposes solubilizing the protein of interest bound to an insoluble structure, easily separable from the soluble proteins of the medium, which method is much more efficient than the methods used until now since it is based on a single pass of precipitation. The method further allows lowering the costs with respect to the methodologies most used currently, mainly based on chromatography methods, especially those based on the purification by means of affinity chromatography. Furthermore, by placing the starch binding domain in the amino end region of the fusion protein preceded by the transit peptide, whereas the polypeptide / protein of interest is in the carboxyl end, it is assured that the protein of interest has not suffered amino acid excisions during the translocation to the plastid, the probability that its amino acid sequence is integral forming part of the starch-granule bound fusion protein being greater, when the purification process from the transgenic plant or extract thereof starts. This is outlined in Figure 2 (wherein the starch binding domain and the transit peptide form part of the natural form of a plant protein which is bound to the starch, the GBSS) thus, as in Figure 3, corresponding to an embodiment in which the starch binding domain is that of a bacterial amylase, embodiment in which the fusion protein is initially synthesized in the form of precursor protein having a transit peptide directing the fusion protein to the plastid and, therefore, to the starch granule. In any of the embodiments, the careful election of the fragment containing the protease recognition site will finally allow obtaining the protein of interest which is not only integral but lacks additional amino acids which will enable its use in sectors as demanding as the pharmaceutical sector.

Within the scope of the method of the invention are the embodiments comprising the prior steps in which the transgenic plant expressing the fusion protein containing the polypeptide of interest is obtained and cultured. The invention also relates to the vector from which a transgenic plant suitable for carrying out the method of the invention can be obtained, to a host organism which contain said vector, as well as the vegetable cells transformed with the vector and the transgenic plants obtained from the same.

Consequently, a first aspect of the invention relates to a method for obtaining purified recombinant proteins from a transgenic plant or transgenic cell culture comprising the following steps:
a) homogenizing the cell culture or a starch-accumulating plant tissue to which is associated a fusion protein characterized in that it comprises:
   i) in the carboxyl end region, the polypeptide corresponding to the recombinant protein to be purified,
   ii) in the amino end region, a starch binding domain,
   iii) a linker fragment containing the protease cleavage site, linker fragment which is located between the amino end region and the carboxyl end region;
b) centrifuging and selecting the most dense fraction containing the starch granule proteins;
c) treating the fraction selected in step b) with the protease which recognized the cleavage site comprised in the linker fragment;
d) centrifuging and selecting the supernatant;
e) optionally, purifying in high grade the recombinant protein contained in the supernatant.

In the event of performing the additional step of purification, it will preformed by any method known by the person skilled in the art, such as any size separation technique, immunoabsorption technique, ion exchange technique or fluorescence detection technique.

Such as has been mentioned, an embodiment of the method of the invention included in the scope thereof is that comprising the steps by means of which the transgenic plant is obtained and cultured. Therefore, in a possible embodiment, the method of the invention comprises the prior steps of:
a) i) obtaining a transgenic plant by means of transforming a wild-type plant with an expression vector comprising a plant expression promoter operatively bound to a coding sequence comprising, in 5'-3' direction, a peptide directing the transition of cytosol to plastids, a starch binding domain, a protease cleavage site and the polypeptide corresponding to the recombinant protein to be purified;
a) ii) culturing the transgenic plant obtained in the previous step in conditions suitable for its growth.

Such as has been previously discussed, those in which the starch binding domain is a bacterial amylase binding domain, as well as those in which the binding domain belongs to a vegetable protein generally an enzyme which is naturally bound to the starch are possible embodiments of the invention. In this second case, it is preferred that the fusion protein comprises the complete polypeptide sequence of said vegetable protein and that it is synthesized in the cytosol in the form of a precursor which comprises the complete amino acid sequence of the precursor form of said vegetable protein, i.e., a precursor which comprises the natural transit peptide of said protein, therefore the coding sequence of the fusion protein will not need any transit peptide in addition to that which is typical of said protein.

In the event that the starch binding domain belongs to a bacterial amylase, said domain can be selected from any of the known SBDs, such as for example those which are found in Figure 1 of the Janecek and Sevcik article (Janacek and Sevcik, 1999). Those which are mentioned in Figure 1 of application WO 00/77165, i.e., those having the sequences represented by SEQ ID NO:3, SEQ ID NO:4, SEQ ID NO:5 and SEQ ID NO:6, for example, can be selected as minimal sequences. In that case, the coding sequence of the fusion protein does need to contain, in 5' with respect to the coding sequence of the SBD, the coding fragment of a transit peptide.

As has been discussed, the invention also relates to the vector by means of which the transgenic plants are obtained, as well as to the host transformed with the same, to the intermediate vectors which allow obtaining the plant transformation vector and the hosts transformed with them.

Thus, one aspect of the invention relates to a vector for use in the method of the present invention comprising, in 5'-3' direction, a promoter allowing the expression in plants to which there is operatively bound the coding sequence of the fusion protein comprising, in the amino to carboxyl direction, a cytosol-plastid transit peptide, a starch binding domain, a linker fragment containing the protease cleavage site and the polypeptide corresponding to the recombinant protein to be purified following the method of the invention. Preferably, said vector is a plasmid.

Similarly, another aspect of the invention is an organism transformed with the above vector. Preferably, the organism is the bacteria *Agrobacterium tumefaciens.*

One more aspect is a transformed vegetable cell with the above host organism. Similarly, one more aspect of the invention is a transgenic plant, its seeds or propagation material, obtained from the above vector and/or the cells of which are transformed with said organism.

Finally, it is also an aspect of the invention the intermediate plasmids from which the transformation vector can be obtained since they will be the plasmids which will enable applying the method of the invention for amplifying and purifying very different proteins. Particularly, in the examples of the present invention, the Gateway® technology of Invitrogen is resorted to preventing the use of endonuclease digestions and ligations, substituting them with consecutive recombination events in the presence of recombinases which recognize pairs of complementary recombinant sites, such as described in the Gateway^{®} Technology protocol: (http://www.invitrogen.com/site/us/in/home/Products-and-Services/Applications/Cloning/Gateway-Cloning/GatewayC-Misc/Protocols.html#bp).
In the method of the invention, the GBSS was used as an example of starch-granule bound protein. To obtain the transformation vector, an intermediate vector which allows fusing the coding sequence of any protein of interest with GBSS by means of the GATEWAY^{®} technology, was used. Therefore, an additional aspect of the invention describes a vector specifically designed for use in the method of the invention, comprising, in 5'-3' direction, a promoter, a GBSS encoding sequence and a nucleotide sequence flanked by attR1 and attR2 sequences which allows cloning any nucleotide sequence flanked by attL1 and attL2 sequences downstream from *GBSS.*

The invention also relates to a host organism, specifically *E. coli,* housing the vector previously described.

### Description of the Drawings

Figure 1 shows a diagram of the synthesis in cytosol, translocation to plastid (stroma), binding to starch, release from the starch granule and purification of a recombinant polypeptide synthesized as part of a fusion protein in which the starch-encapsulating region (SER: starch encapsulating domain) is located in the carboxyl fragment of the fusion protein, whereas the polypeptide of interest (herein depicted by the green fluorescent protein, GFP) is located in the amino area following the transit peptide (TP). GFP and SER are separated by a linking amino acid sequence specifically recognized by an endoprotease. The translocation to the stroma by means of the toc-tic system involves excising an undetermined number of amino acids (modification represented by means of the end of irregular arrow) which can cause the GFP protein to loss part of its amino acids from the amino end or to keep a portion of the transit peptide, which modification is maintained once the polypeptide is released and purified.
Figure 2 shows the diagram of the synthesis in the cytosol, translocation to plastid (stroma), binding to starch, release from the starch granule and purification of a recombinant polypeptide synthesized as part of a fusion protein in which the polypeptide of interest (herein depicted by the green fluorescent protein, GFP) is localized in the carboxyl region of the fusion protein, whereas in the amino area there is located a vegetable protein having a starch binding domain (herein depicted by the GBSS: granule-bound starch synthase) having in its precursor form its own transit peptide (TP). The translocation to stroma by means of the toc-tic system does not affect the GFP protein which is recovered intact once the polypeptide is digested with the endoprotease (in this case, enterokinase) and purified.
Figure 3 shows the diagram of the synthesis in the cytosol, translocation to plastid (stroma), binding to starch, release from the starch granule and purification of a recombinant polypeptide synthesized as part of a fusion protein in which there is a starch binding domain (SBD) of bacterial origin which is located in the amino fragment of the fusion protein, preceded by the transit peptide (TP), whereas the polypeptide of interest (herein depicted by the green fluorescent protein, GFP) is located in the carboxyl area. The translocation to stroma by means of the toc-tic system can affect the SBD, although it should not be extended to the GFP which, again, would be recovered intact once the polypeptide corresponding to the GFP is digested with the endoprotease (in this case, enterokinase) and purified.

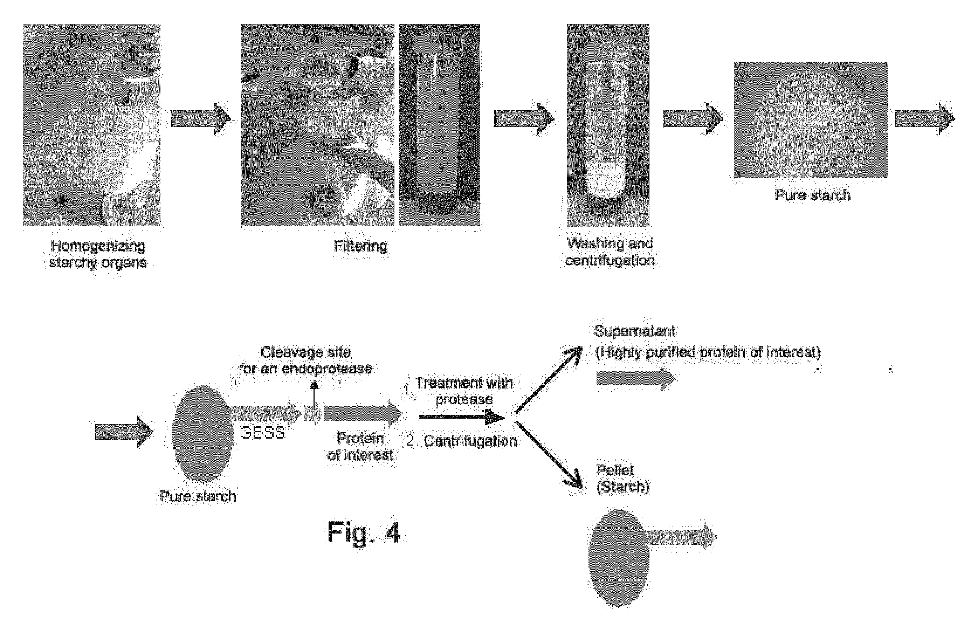

Figure 4 shows the steps for obtaining a polypeptide of interest (recombinant protein) following the method of the present invention from potato tubers expressing the protein of interest bound to the GBSS: homogenizing the tubers, filtering, washing and centrifuging, obtaining the pure starch granules, digesting with the corresponding protease, centrifuging again and separating the protein of interest in the supernatant while the starch granules remain in the pellet.

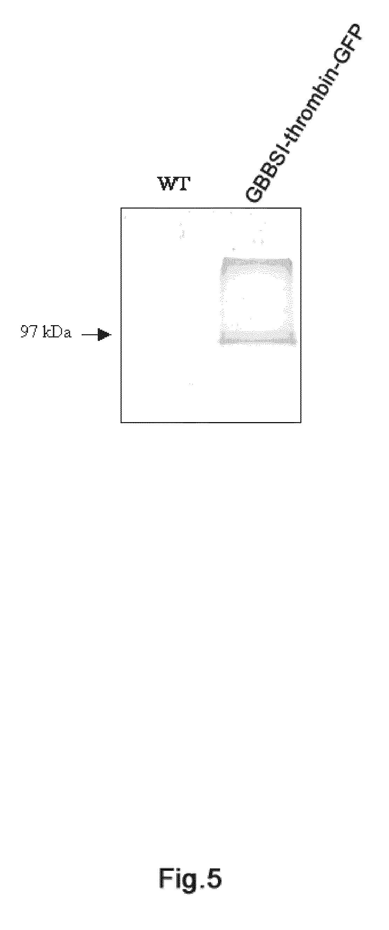

Figure 5 shows the detection by means of anti-GFP immunoblotting of the GBSS-thrombin-GFP protein in GBSS-thr-GFP-expressing transgenic plants of *N. benthamiana.*

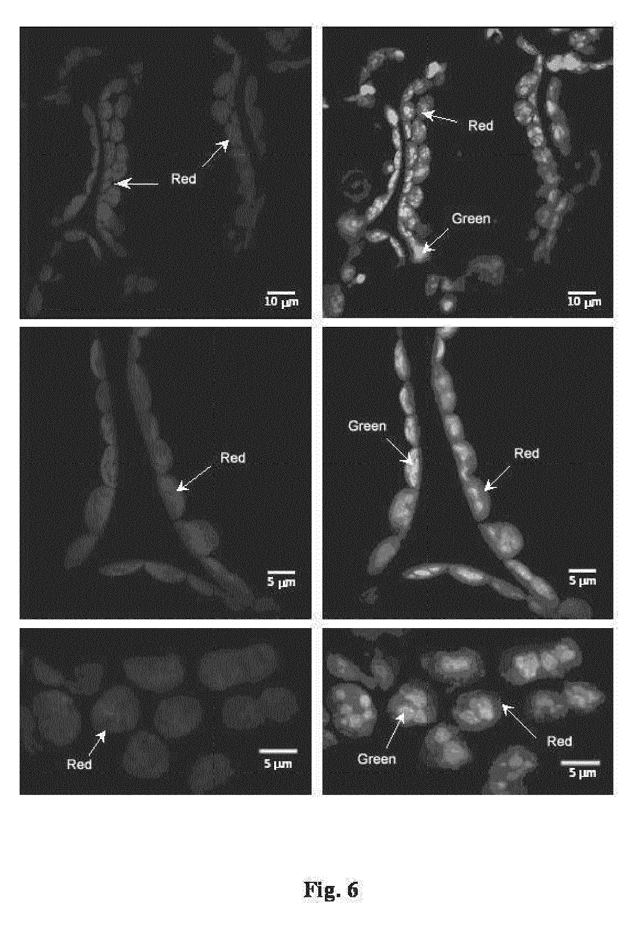

Figure 6 shows the sub-cellular localization of GBSS-thrombin-GFP. The photographs show the fluorescence in Arabidopsis plant cells expressing *GBSS-thr-GFP* which have been subjected to analysis by means of a D-Eclipse C1 (NIKON) confocal microscope equipped with an excitation laser Ar 488, a filter BA515/30 for green emission and a filter BA650LP for red emission. It can be observed in the photographs that the green fluorescence emitted by GBSS-thrombin-GFP is associated to the starch granules, thus confirming the binding of the GFP to the protein GBSS. The red fluorescence is emitted by the chlorophyll existing in the plastids.

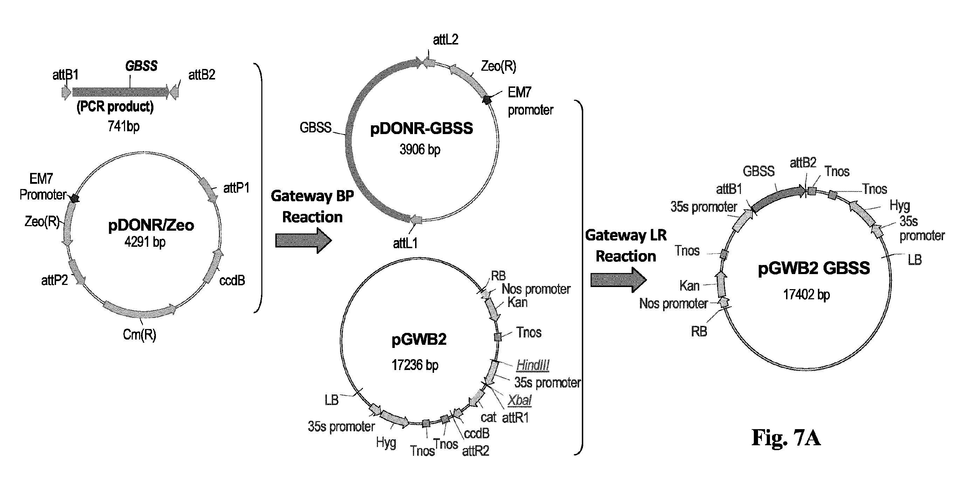

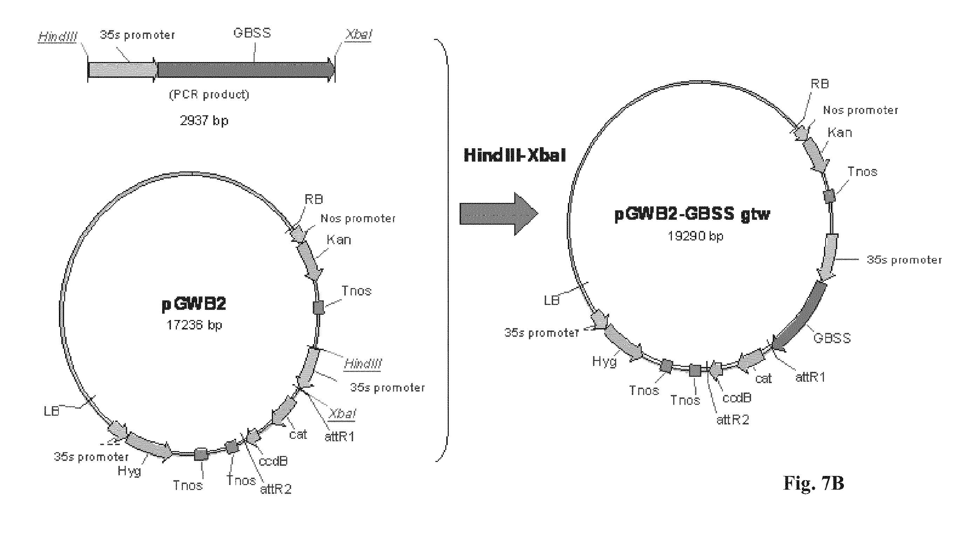

Figure 7 shows the steps for constructing the plasmid pGWB2-GBSS gtw, allowing the introduction of the coding sequence of any protein of interest bound to that of the GBSS.
Figure 7A shows the generation by means of GATEWAY technology the pGWB2-GBSS construct containing the GBSS gene under the control of the 35S promoter.
Figure 7B shows the generation of the plasmid pGWB2-GBSS gtw incorporating the GATEWAY recombination cassette.

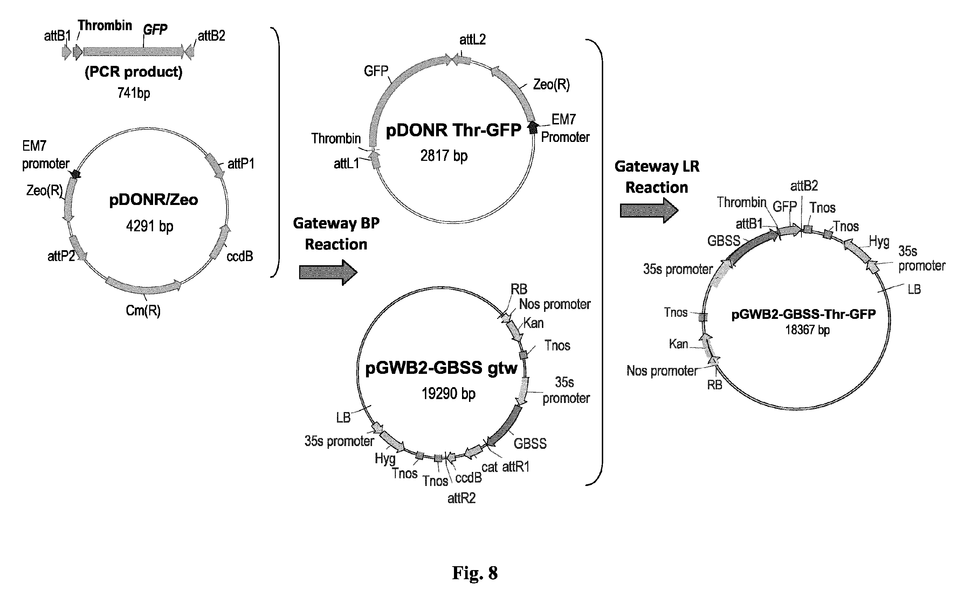

Figure 8 shows the steps for constructing the plasmid pGWB2-GBSS-Thr-GFP, needed for producing plants through A. *tumefaciens* ectopically expressing the GBSS-bound GFP through an amino acid sequence recognized by an endoprotease, in this case the thrombin. Introduction of the Thr-GFP sequence by means of GATEWAY technology in the vector pGWB2-GBSS gtw of Figure 7.

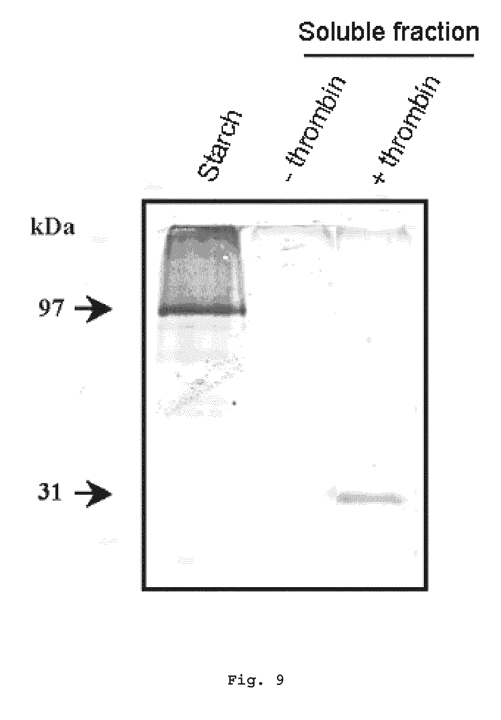

Figure 9 shows the analysis by means of immunoblotting of the GFP protein using anti-GFP antibodies. The purified starch granules of transgenic plants expressing *GBSS-Thr-GFP* were subjected to treatment with or without the protease thrombin. Thereafter, they were subjected to a pass of centrifugation at 1,000 g for 10 minutes. From left to right: starch granules and supernatants obtained after centrifuging starch granules not treated and treated with thrombin, respectively.

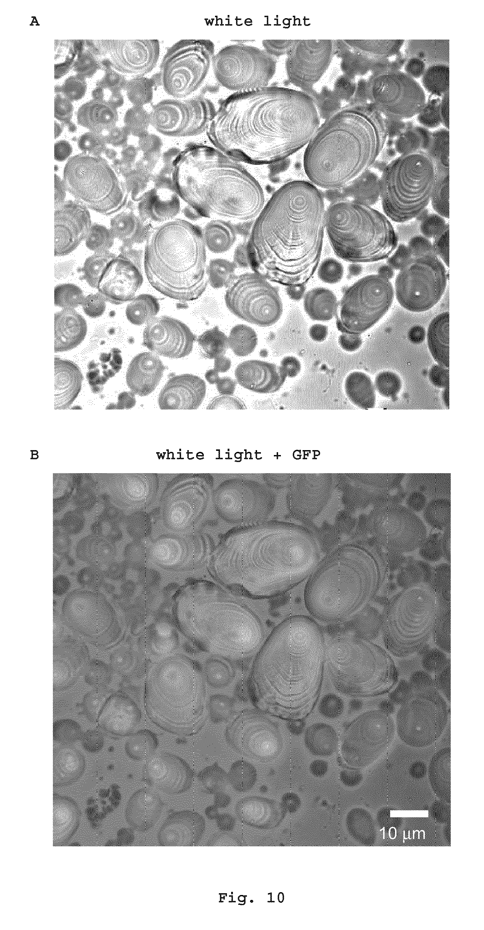

Figure 10 shows the Confocal microscopy photographs of potato tuber starch granules expressing a fusion protein with GBSS- thrombin recognition sequence - GFP. The photograph below shows green fluorescence in all the granules and in all the surface thereof, indicating the presence of the GFP in active form.

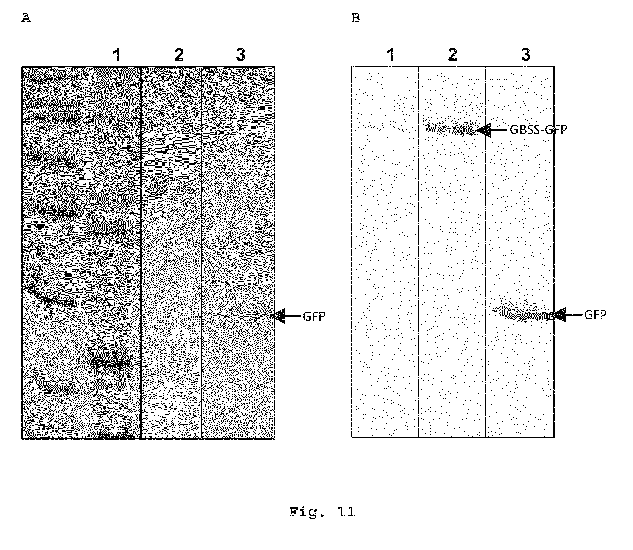

Figure 11 shows the verification of the purity and concentration of the GFP isolated from starch granules which expressed the fusion protein between the GBSS and the GFP. Figure 11A: shows the SDS-PAGE gel electrophoresis (polyacrylamide with SDS) and staining with Coomasie Blue. Figure 11B shows the photograph after Western blot. Lanes: (1): potato tuber protein extract which expressed GFP bound to the GBSS by means of a linker fragment with the thrombin recognition sequence. (2) protein extract of starch purified from the same plant. (3) protein released from the starch after digestion with thrombin and centrifugation. The positions corresponding to the GBSS-GFP fusion protein and the free GFP are indicated with arrows.

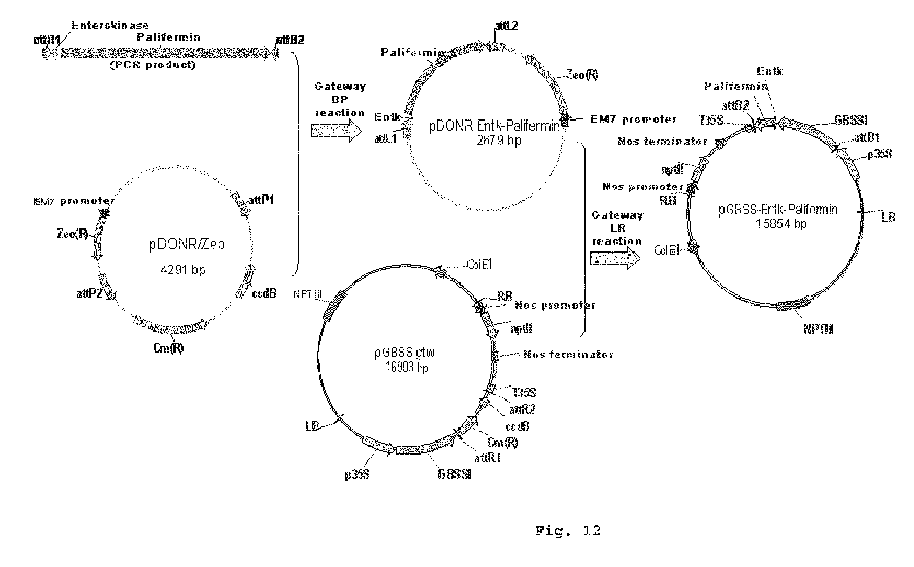

Figure 12 shows the steps for constructing by means of GATEWAY technology, the plasmid pGBSS-Entk-Palifermin, allowing the generation of transgenic plants expressing a fusion protein in which the palifermin coding sequence is bound to the GBSSI by means of a linker fragment with the enterokinase recognition sequence. A PCR product corresponding to the palifermin coding sequence associated to the enterokinase recognition coding sequence is generated, all the fragment being flanked by attB1 and attB2 sequences. The fragment is inserted into the plasmid pDONR/Zeo by means of a recombination reaction in the presence of the corresponding recombinase, giving rise to the plasmid pDONR Entk-Palifermin. A new recombination reaction with a plasmid having the GBSS coding sequence followed by a GATEWAY recombination cassette (in this case the plasmid used is pGBSS gtw) bound operatively to the 35S promoter of the cauliflower mosaic virus, allows creating a plasmid (pGBSS-Entk-Palifermin) with the coding sequence of the GBSS-palifermin fusion protein, separated by the enterokinase recognition sequence, all the coding sequence being under the control of the 35S promoter.

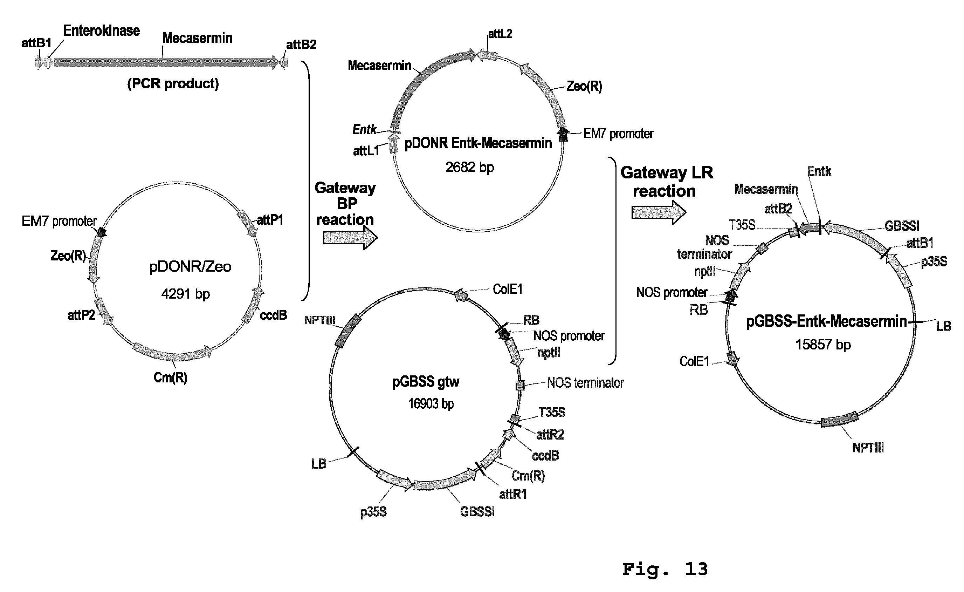

Figure 13 shows the steps for constructing by means of GATEWAY technology, the plasmid pGBSS-Entk-Mecasermin, allowing the generation of transgenic plants expressing a fusion protein in which the mecasermin is bound to the GBSS by means of a linker fragment with the enterokinase recognition sequence, following a method similar to that of Figure 12.

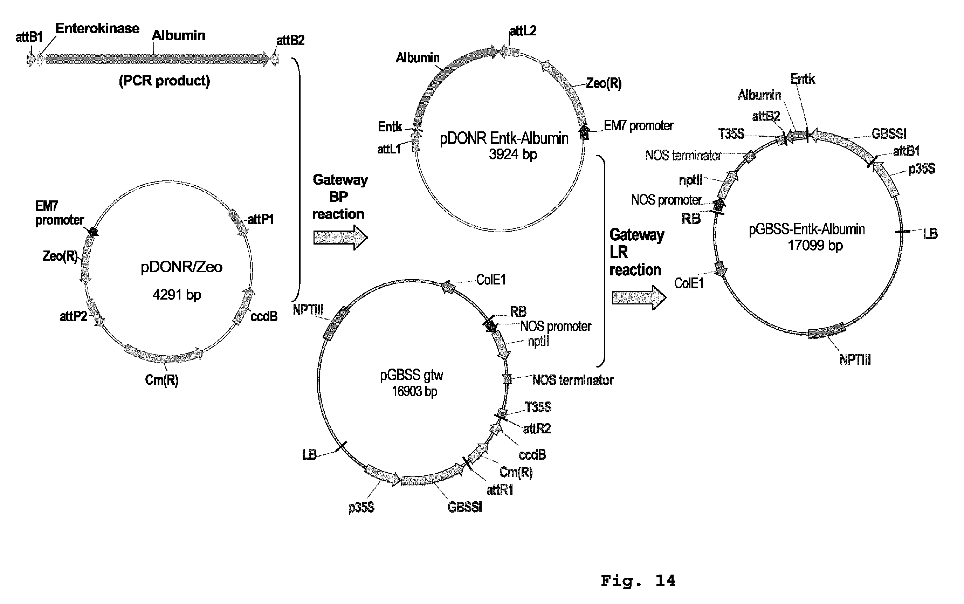

Figure 14 shows the steps for constructing by means of GATEWAY technology, the plasmid pGBSSI-Entk-Albumin, allowing the generation of transgenic plants expressing a fusion protein in which the mature albumin is bound to the GBSSI by means of a linker fragment with the enterokinase recognition sequence, following a method similar to that of Figure 12.

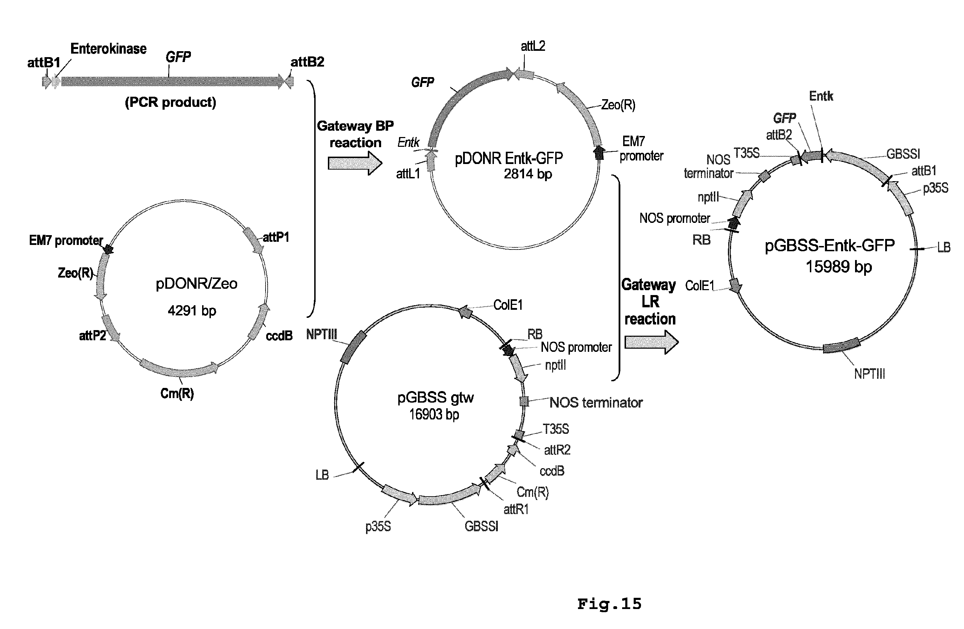

Figure 15 shows the steps for constructing by means of GATEWAY technology, the plasmid pGBSS-Entk-GFP, allowing the generation of transgenic plants expressing a fusion protein in which the GFP is bound to the GBSS by means of a linker fragment with the enterokinase recognition sequence, following a method similar to that of Figure 12.

### Detailed Description of the Invention

Such as has been previously mentioned, the present invention provides an alternative for the large scale purification of recombinant proteins produced in transgenic plants or transgenic plant culture expressing the recombinant protein to be purified.

The new method for purifying proteins is based on the production of starch-accumulating plants or cells which express proteins of interest fused with proteins for the purpose of binding to the starch granule such as the GBSS (Granule Bound Starch Synthase), or with starch-binding domains of bacterial enzymes, through a linker fragment containing a sequence specifically recognized by endoproteases such as thrombin or enterokinase. Thus the protein of interest is anchored to the starch granule, the high density of which allows an easy purification from the rest of the cellular components after a single pass of tissue homogenization, centrifugation and solubilization of the recombinant protein of interest after a digestion treatment with endoprotease. The method is exemplified in Figure 4. As can be seen, it is a simple more economical method than the chromatography methods which are commonly used for purifying recombinant proteins (especially the fusion proteins), particularly if it is compared with the affinity columns which are used often. The time, furthermore, is less than one day: in the specific case of Example 3 which is presented below, 12 hours were enough for obtaining 1 mg of highly purified GFP (example, in this case, of recombinant protein of interest to be purified) from 10 kg of potato tubers.

The fact that the fusion protein is initially isolated bound to the starch granule, it has the additional advantage of preventing the risks which involve the association with, for example, oleosins, which sometimes had contaminations with possible allergenic effect. Therefore, the association to the starch granule is an additional advantage for purifying proteins which are to be used supplied to human beings.

This method has been worked on using potato plants expressing the green fluorescent protein (GFP) fused to the carboxyl terminal end of the GBSS through an amino acid sequence specifically recognized by an endoprotease. It is very important, as previously discussed, that the polypeptide of interest to be purified binds to the carboxyl terminal end of the fusion protein fragment which comprises the starch binding domain because, otherwise, as outlined in Figure 1, the actuation of the toc-tic system for translocating the cytosol to plastid could cause obtaining the polypeptide of interest at the end with the absence of part of its sequence, or with additional amino acids. Since it is not an integral sequence, the polypeptide could not be used as such in pharmaceutical applications or applications of other type in which the integrity of the amino acid sequence of the protein of interest is required. This condition with respect to the organization of the elements in the fusion protein involves a significant difference with respect to the systems suggested in the applications WO 98/14601 and WO 00/77165, especially the first, wherein the granule-binding element was preferably located in the carboxyl end region. This condition with respect to the organization of the elements in the fusion protein also enables using the method in an actual manner for purifying proteins, having assurances that the same will finally be isolated not only integrally, but being able to predict whether the final purified polypeptide may or may not contain any additional amino acid in any of its ends.

As can be seen in Examples 2 and 3, the pure recombinant starch treated with the suitable endoprotease releases highly purified GFP. Therefore, the main challenge of this method for producing and purifying recombinant protein resides in obtaining pure starch.

It is also important to indicate that, as shown in Figure 6 and Figure 10, the GFP remains active in the starch granule which is bound the fusion protein, therefore this new method allows producing and purifying properly folded and assembled proteins from starch obtained from storage organs.

The system used in the examples which are presented in the following application, based on the use of the 35S promoter, allows GFP accumulation levels of approximately 6 mg per kg of fresh tuber weight (approximately 60 mg of GFP per kg of potato tuber starch). As can be verified in Example 3, in a single pass, the new method allows purifying 600-1000 times the GFP obtaining a minimum of 50-100 ng of highly purified GFP from 1 kg of transgenic potato tuber in a simple and fast manner. It is, therefore, an efficient, fast and simple method involving an interesting alternative to the expensive methodologies currently used.

As discussed in Example 3, the expression system based on the use of the cauliflower mosaic virus 35S promoter (CaMV) allows GFP accumulation levels of approximately 6 mg per kg of fresh tuber. It is estimated that the GFP involves approximately 0.1% of the total protein of the transgenic tubers. The system can be optimized with embodiments included within the scope of the invention, in which stronger promoters which allow increasing the accumulation levels of the polypeptide of interest are used.

Another interesting possible embodiment of the method of the invention for increasing the yield of the finally obtained purified protein is the application of an additional step, prior to digesting the starch granules with the endoprotease, step in which treatments breaking or digesting the starch granule are applied, such that the endoproteases have access to most of the recombinant protein of interest bound to the granule and not only to that existing in the surface of the starch granule. It is a particularly interesting embodiment because, from approximately 6 mg of GFP existing in 1 kg of tuber, only 1% (50-100 ng of GFP specifically) are extracted by means of a single digestion treatment of the native and intact starch granule. A possibility is the use of enzymatic digestion, for example, by means of amylases, although physical rupturing treatment breaking up the starch granules such as fragmentation or crushing can also be used.

Many variations can be made in the method of the invention without modifying its basic elements, all therefore being included within the scope of the invention.

On one hand, as has been discussed, the starch binding domain of a bacterial enzyme having such domain can be used as the starch binding domain which tends to be referred to, in an abbreviated form, as SBD. As has been discussed above, there is many literature in which examples of bacterial SBDs are found. In the embodiments which include SBDs of bacterial origin, it is important to take into account that, the translocation of the fusion protein to plastid will require the presence of a transit peptide in the precursor fusion protein synthesized in the cytosol. Therefore, the expression vector whereby the transgenic plant is prepared must have, as part of the coding sequence of the fusion protein, the coding part corresponding to a suitable transit peptide. The transit peptide the sequence of which is represented by SEQ ID NO:8 can, for example, be used, using the sequence represented by SEQ ID NO:7 as the possible coding sequence thereof. It is a peptide used in the above occasions by the group of the present inventors, its usefulness for sending proteins such as the green fluorescent protein (GFP) (Bahaji et al., 2011a) or bacterial proteins such as the GlgC (Bahaji et al., 2011b) to the plastid has been verified. It is the transit peptide which, in nature, allows incorporating the protein P541 inside the capsicum chromoplast (Houlne *et al.,* 1994).

In the event that the starch binding domain belongs to a vegetable protein, it is preferred that the fusion protein comprises the complete polypeptide sequence of said vegetable protein and that it is synthesized in the cytosol in the form of a precursor which comprises the complete amino acid sequence of the precursor form of said vegetable protein, i.e., a precursor which comprises the natural transit peptide of said protein. Thus, the coding sequence of the fusion protein will comprise the complete natural coding sequence of the selected starch binding vegetable protein, coding sequence which will include its natural peptide precursor, an additional transit peptide no being needed prior to said protein.

Although it is not essential for the proposals of the invention, the presence of the natural complete sequence of a vegetable protein which binds to the starch is preferred to the presence of the fragments thereof because it ensures that the amino acids removed by the toc-tic system in the process of translocation to plastid will belong to said protein, neither the protease recognition sequence which will be used to separate the polypeptide of interest from the fusion protein nor said fusion protein are being affected.

The vegetable protein comprising a starch binding domain can be selected from the group consisting of:
- granule-bound starch synthase (GBSS) (EC 2.4.1.242),
- glucan-dikinase (R1) (EC 2.7.9.4)
- one of the starch-branching enzymes from the group consisting of BEI, BEIIa, BEIIb, (EC 2.4.1.18),
- one of the soluble starch synthases from the group consisting of SSI, SSII, SSIII (EC 2.4.1.21),
- and the plastidial starch phosphorylase (EC 2.4.1.1).

The coding sequence of said vegetable protein can be identical to a natural sequence or can be a sequence having at least 60% homology with a natural sequence, provided that the protein synthesized from said coding sequence maintains the capacity of binding to the starch and presenting a signaling sequence capable of acting as transit peptide directing the precursor initially synthesized to the plastid. Preferred embodiments of the invention are those which resorted to the natural sequence of any of the previous enzymes as occurs, for example, in Examples 1 and 2 of the present application, wherein, the natural sequence of the *Arabidopsis thaliana* GBSS which is encoded by the gene *At1g32900* has been used after cloning it. The natural sequence of *Solanum tuberosum,* for example (accession no. in UniProt: Q00775; accession nu. in EMBL to the coding sequence: CAA41359.1), can also be used.

When the natural sequence of a vegetable protein with starch binding domain is used, resorting to the sequence corresponding to the species from which the transgenic plant is obtained is preferred although it is not essential.

With respect to linker fragment having a cleavage site recognized by a protease, the protease could, in principle, be any protease. However, if wanting to obtain the intact peptide of interest at the end of the process, the cleavage site must be chosen such that it is also not present in the internal sequence of the protein of interest. A possible protease to be considered is thrombin (which cleaves the polypeptide sequences after the amino acid arginine, Arg, the recognition sequences being considered optimum for cleaving are Gly-Arg//Gly and A-B-Pro-Arg-//-X-Y, wherein A and B are hydrophobic amino acids and X and Y are non-acidic amino acids). When the polypeptide of interest is intended for pharmaceutical use, a protease the recognition sequence of which remains entirely in the carboxyl end after the cleaving, no amino acid remaining in the amino end which may involve a modification to the sequence of the polypeptide of interest is particularly preferred. An example could be the enterokinase, a specific protease which performs a cleavage after the amino acid lysine when it finds the Asp-Asp-Asp-Asp-Lys sequence; thus, the amino acid after which the cleavage is performed is the final amino acid of its recognition sequence, of which no residue remains in the amino end generated after the cleavage.

The option of using the enterokinase and its recognition sequence have especial interest, as has been discussed, when the polypeptide of interest is to be used in the pharmaceutical sector or in any other sector in which the total integrity of the amino acid sequence of the recombinant protein is required, since it is an assurance that the polypeptide obtained at the end of the method will have its integral sequence without vestiges of the enzyme cleavage sequence and without lacking part of its amino acids. It is for this reason that it is the chosen endoprotease in Example 4 referring to the use of the method of the invention for proteins of clinical or therapeutic interest, such as palifermin, mecasermin and albumin. The method in which some of said proteins is the polypeptide of interest to be amplified and purified and the vectors of the invention comprising the coding sequence of fusion proteins which form part said polypeptides, are comprised within the scope of the present invention.

As discussed below, the system has been developed using the GFP as the polypeptide of interest model (the recombinant protein to be amplified and purified) due to the fact that it is a protein which emits green fluorescence the expression of which is therefore easy to be determined, allowing an easy visualization of the results. Since it is a common test tool, finding data about its characteristics and reagents useful for working with it, such as specific antibodies, is easy. Given its interest, a specific embodiment of the method of the invention of special interest is considered as that in which the GFP acts as a polypeptide of interest, the linker fragment being able to be one which has a thrombin cleavage site and the vegetable protein, GBSS, such as in Examples 1, 2 and 3 of the present application. The vectors of the present invention having the coding sequence (SEQ ID NO:1) of the fusion protein corresponding (SEQ ID NO:2) to that GBSS-thrombin cleavage site -GFP fusion also have special interest.

With respect to the species from which the transgenic plant is obtained, any plant could be used in principle, although the plant is preferred to be starch-accumulating plant such as, for example, a cereal plant such as rice, barley, wheat and corn, potato, tobacco, tomato or *Arabidopsis thaliana* plants. Assays performed with *Nicotiana benthamiana* (one of the species of the tobacco plant) or *Solanum tuberosum* (potato) are shown in the present application, but the technical effects shown in the present application can be extrapolated to any starch-accumulating plant as well as to any organ of the starch-accumulating plant (tubers, leaves, fruits, seeds...).

Obtaining the transgenic plant used in the method of the present invention requires the transformation with expression vectors specifically designed for the proposals of the invention. Thus, one aspect of the invention relates to an expression vector for transforming plants comprising, in 5'-3' direction, a promoter allowing the expression in plants, to which there is operatively bound the coding sequence of the fusion protein comprising, in the amino to carboxyl direction, a cytosol-plastid transit peptide, a starch binding domain, a linker fragment containing the protease cleavage site and the polypeptide corresponding to the recombinant protein to be purified following the method of the invention. Preferably, said vector is a plasmid.

All the possible alternatives and preferences previously mentioned with respect to the elements making up the fusion protein, its coding sequence and the promoter from which they are expressed are also applicable to the vectors of the invention by means of which the transgenic plant is generated. The specific vectors corresponding to the alternatives used in the examples presented below in the present application are significant embodiments of said aspect of the invention:
- the vectors in which the promoter to which there is operatively bound the coding sequence of the fusion protein is the 35S promoter,
- the vectors in which the cytosol-plastid transit peptide sequence and the starch binding domain are comprised within the natural coding sequence of a vegetable protein which is bound to the starch, with especial preference for the GBSS (very particularly that of *Arabidopsis thaliana* or the GBSSI of *Solanum tuberosum);*
- the vectors in which the linker fragment comprises the coding sequence of thrombin or enterokinase recognition site;
- the vectors comprising the coding sequence of a polypeptide selected from the group consisting of GFP, palifermin, mecasermin and mature human albumin.

For operation, it is also preferred that the vector has been obtained using the GATEWAY^{®} technology. For that reason, preference in that the coding sequence of the fusion protein is flanked by aatB: attB1 and attB2 sequences, is also obtained.

The GATEWAY^{®} technology considerably simplifies the cloning method, however, the expression vector used in the method of the invention can be obtained by means of any other known cloning strategy, e.g.: cleaving and folding by means of restriction enzymes, ligases, and any other used in the sector.

The embodiments of the method of the invention in which the transgenic plant is obtained by means of one of said vectors of the invention are also comprised within the scope of the invention.

The host organism including said vector is also an important part of the invention both because it will allow the amplification thereof and because of its possible intermediary role in obtaining the transgenic plant. This is why the bacterial host in which said vector is introduced is also an aspect of the invention. For that same reason, the host is preferred to be *Agrobacterium tumefaciens,* since it is the suitable route to introduce the construct comprising the coding sequence of the fusion protein and the promoter in a plant.

Additional aspects of the invention describe the vegetable cells transformed with the mentioned host and the transgenic plants formed by said cells or obtained therefrom. The transformed cells expressing the fusion protein bound to starch granule could also be used to carry out the method of the invention, purifying the protein from the cultures thereof. Cultures from other cell lines of plants transformed with a vector of the invention which can be cultured by methods well known by the persons skilled in the art, preferably fast growing cell lines such as the cell line derived from tobacco plants BY-2, preferably cultured in the presence of cytokines (Enami *et al.,* 2011), could also be used. The plant cells are commonly cultured as cells in suspension in liquid medium or also in solid medium in the form of callus (non-differentiated cell mass). In the case of non-differentiated cells, it is necessary to obtain the necessary equilibrium between auxins and cytokines. For more detailed information, literature about the vegetable cell culture in general (Bhojwani and Razdan, 1996; Georgiev *et al.,* 2009; Singh and Seema, 2009), or about the BY-2 cells in particular (Nagaka *et al.,* 2004) can be consulted.

Similarly, said transgenic plants comprised in the scope of the invention, can be defined as those transgenic plants which comprise, integrated in their genome, a DNA fragment which comprises a promoter for the expression in plants, to which there is operatively bound the coding sequence of the fusion protein of the invention. Therefore, a transgenic plant, its seeds or propagation material is also included within the scope of the invention, characterized in that its genome has integrated therein, in 5'-3' direction, a promoter allowing the expression in plants, to which there is operatively bound the coding sequence of the fusion protein comprising, in the amino to carboxyl direction, a cytosol-plastid transit peptide, a starch binding domain, a linker fragment containing the protease cleavage site and the polypeptide sequence of a protein not naturally expressed in the species to which the transgenic plant belongs. The last protein will logically be the recombinant protein to be amplified and purified.

For the purposes of the present invention, the following terms are set forth:
- Transgenic plant: plant the genome of which has been modified by means of genetic engineering with the objective of achieving biological characteristics different and/or improved with respect to that of the wild-type control plant (WT).
- Transformed vegetable cells: are vegetable cells having a genetic alteration resulting from the introduction and expression of external genetic material in their genome.
- Ectopic expression of the chimeric protein GBSS-thrombin-GFP: a plant ectopically expresses the chimeric protein GBSS-thrombin-GFP when an anti-GFP immunoblotting detects a band in a transformed plant extract which is not detected in a WT plant extract cultured in the same conditions and at the same time. By way of illustration, it can be observed in Figure 5 that in a protein extract of transgenic *Nicotiana benthamiana* plants expressing *GBSS-thr-GFP* a bands having the size corresponding to the chimeric protein which is not detected in WT plant extracts is detected using a GFP specific antibody. Another method for confirming that a plant expresses the protein GBSS-thrombin-GFP is by means of detecting the fluorescence emitted by the GFP protein. As shown in Figure 6, the fluorescence is localized only and exclusively in the starch granule, confirming that the GFP is bound to the GBSS.

In the examples which are presented below in the present application, the GBSS was used as the example of protein bound to the starch granule, specifically the GBSS of *Arabidopsis thaliana.* To obtain the transformation vector, intermediate vectors designed specifically for use in said method, obtained by means of the GATEWAY^{®} technology were used in which the coding sequence of the GBSS is already operatively bound to the chosen promoter (in this case, the 35S), vectors which allow fusing the coding sequence of any protein of interest with the GBSS. Said vectors, therefore, involves a rather useful tool when applying the method of the invention to any protein, involving a useful intermediate to which it is only necessary to add the coding sequence of the specific polypeptide of interest at each time. Therefore, another aspect of the invention is formed by an expression vector comprising, in 5'-3' direction, a promoter allowing the expression in plants, to which there is operatively bound the coding sequence of a vegetable protein which is bound to the starch, coding sequence which is followed by a fragment flanked by attR1 and attR2 sequences, which fragment preferably contains an indicator gene or a selection gene. This allows cloning any nucleotide sequence flanked by attL1 and attL2 sequences downstream from the coding sequence of the vegetable protein with a starch binding domain. Said vector, could include any promoter commonly used for such purpose, as well as any suitable terminator, and different antibody-resistance markers without altering the essence of the invention. Preferably, said vector is a plasmid. Preferably, the encoded vegetable protein is the GBSS with especial preference for that of *Arabidopsis thaliana* or that of *Solanum tuberosum.* A possibility is that the vector is, specifically, a vector comprising, in 5'-3' direction, the 35S promoter, a sequence encoding the GBSS and a nucleotide sequence flanked by attR1 and attR2 sequences, such as the vector *pGWB2-GBSS gtw,* described in detail in Example 1 and in Figure 7, and also the vector *pGBSS gtw* depicted in Figures 12-15 and used in Example 4.

A bacterial host transformed with said vector also forms an additional aspect of the invention. The most preferred case is that in which the host is *E. coli.*

The following examples illustrate in detail the invention. They must not be interpreted as limiting the scope of the invention.

### EXAMPLES

The examples are proceeded to be described below, in which the method for obtaining the transgenic plants expressing recombinant proteins fused to the protein GBSS through a sequence specifically recognized by a protease, is shown in detail. The fluorescence GFP protein was used as an example of protein bound to the GBSS. The amino acid sequence recognized by the thrombin was initially used as an example of sequence specifically recognized by a protease.

### Example 1: Obtaining and characterizing transgenic plants expressing GBSS-thrombin-GFP

### 1.1. Obtaining the plasmid pGWB2-GBSS gtw

The production of transgenic plants expressing proteins bound to the GBSS through a amino acid sequence recognized specifically by proteases requires the prior production of a binary plasmid the steps of preparations of which are illustrated in Figure 7. The GBSS is encoded by the *A*. *thaliana Atlg32900* gene. Oligonucleotides specific for the *GBSS* gene were synthesized from its nucleotide sequence. These oligonucleotides were used to amplify the complete cDNA fragment encoding the GBSS by means of RT-PCR from the entire RNA of *Arabidopsis* leaves. The amplified fragment was cloned in the vector pGEM-T easy (Promega), giving rise to plasmid *pGBSS* which was amplified in the host bacteria XL1 Blue. The cDNA encoding the GBSS flanked by attB recombination sites was amplified from the plasmid *pGBSS* using the oligonucleotides GBSS-attB1 (5'-ggggacaagtttgtacaaaaaagcaggcttaatggcaactgtgactgcttcttc-3') and GBSS-attB2 (5'-ggggaccactttgtacaagaaagctgggtatcacggcgtcgctacgttctc-3'). The PCR generated product was cloned in the vector pDONR/Zeo by means of GATEWAY^{®} recombination with the attP1 and attP2 recombination sites, giving rise to the plasmid *pDONR-GBSS.* By means of a GATEWAY^{®} recombination reaction the *GBSS* gene was transferred from the plasmid *pDONR-GBSS* to the plasmid *pGWB2* generating the *pGWB2-GBSS* construct containing the *GBSS* gene under the control of the 35S promoter. By using the plasmid *pGWB2-GBSS* and the oligonucleotides 35S-HindIII (5'-cgtgctcccaagcttactagagccaagctgatctcc-3') and GBSS-XbaI (5'-ggctctagactcggcgtcgctacgttctcc-3') as template, the *35S-GBSS* sequence (formed by the 35S promoter fused to the GBSS gene flanked by the HindIII and XbaI restriction sites) was amplified by means of PCR. In the plasmid *pGWB2,* the 35S promoter was replaced by the PCR product *35S-GBSS* by means of the HindIII and XbaI restriction sites, the plasmid *pGWB2-GBSS gtw* which comprises the constitutive 35S promoter, the *GBSS* gene and a GATEWAY^{®} recombination cassette flanked by attR1 and attR2 sequences allowing the introduction of any gene of interest to produce a recombinant protein formed by the protein of interest fused to the C-terminal end of the protein GBSS, being created. The vector *pGWB2-GBSS gtw* was introduced by electroporation in *E. coli.*

### 1.2. Obtaining the plasmid pGWB2-GBSS-Thr-GFP

The sequence designed with the name *GFP-Thr* was amplified by PCR from a cDNA encoding the GFP protein using the oligonucleotides Thr-Egfp-attB1 (5'-ggggacaagtttgtacaaaaaagcaggcttaatgctggtgccgcgcggcagcatggtgagcaagggcgaggagc-3') and Egfp-attB2 (5'-ggggaccactttgtacaagaaagctgggtattacttgtacagctcgtccatgc-3'). The oligonucleotide Thr-Egfp-attB1 has the attB1 sequence (5'-ggggacaagtttgtacaaaaaagcaggctta-3-) followed by the sequence (5'-atgctggtgccgcgcggcagc-3) encoding a cleavage site of the protease thrombin and a sequence (5'-catggtgagcaagggcgaggagc-3') encoding the N-terminal end of the protein of interest (in this case the GFP). The oligonucleotide Egfp-attB2 has a nucleotide sequence (5'-ggggaccactttgtacaagaaagctgggta-3') encoding the C-terminal end of the protein of interest (in this case the GFP) followed by the attB2 sequence (5'-ttacttgtacagctcgtccatgc-3'). The PCR product generated was cloned by means of GATEWAY^{®} recombination in the vector pDONR/Zeo, giving rise to the plasmid *pDONR-Thr-GFP.* The plasmid *pGWB2-GBSS-Thr-GFP* which comprises the constitutive promoter 35S, the chimeric *GBSS-thromb-GFP* gene and the terminator Nos was obtained from *pDONR-Thr-GFP* and *pGWB2-GBSS gtw* by GATEWAY^{®} recombination.

### 1.3. Obtaining transgenic plants ectopically expressing_proteins bound to GBSS through a sequence recognized by thrombin

The plasmid *pGWB2-GBSS-Thr-GFP* was introduced by electroporation in *A*. *tumefaciens.* The resulting strain was used to transform *Nicotiana benthamiana, Solanum tuberosum* and *A*. *thaliana* plants according to protocols described in the literature (Rocha-Sosa *et al.* 1989; Clough and Bent 1998; Voinnet *et al.* 1999).

The plasmid *pGWB2-GBSS-Thr-GFP* contains the cassette formed by the *A*. *thaliana* GBSS *gene,* a sequence encoding the cleavage site of the protease thrombin and the gene encoding the GFP. The nucleotide sequence of this expression cassette is represented in SEQ ID NO:1. The deduced amino acid sequence of the protein GBSS-thrombin-GFP is represented in SEQ ID NO:2. Transgenic *N. benthamiana, A. thaliana* and potato plants expressing *GBSS-thr-GFP* in a constitutive manner were obtained by using an *A*. *tumefaciens* strain (which houses the plasmid *pGWB2-GBSS-Thr-GFP*). When they are compared with non-transformed plants, the plants expressing the chimeric protein GBSS-thrombin-GFP accumulate a protein of approximately 96 kDa which is recognized by the specific polyclonal antibody of the GFP which do not appear in non-transformed plants (Figure 5). The transformed plants were subjected to sub-cellular fluorescence localization analysis of GFP by means of a confocal microscope D-Eclipse C1 (NIKON) equipped with a laser with excitation Ar 488, a filter BA515/30 for green emission, a filter BA650LP for red emission and a light detector. It can be observed in the photographs of Figure 6 that the GFP protein is localized in the starch granules.

### Example 2: Purifying and identifying the GFP protein of the transformed plants with the GBSS-thromb-GFP construct

### 2.1. Purifying the GFP protein from transgenic plants

The protocol for purifying the GFP protein from starch granules of plants transformed with *35S-GBSS-thr-GFP* is described below:
- 1 g of plant leaf transformed with *35S-GBSS-thr-GFP* was homogenized with a beater in 10 ml of extraction buffer (50 mM Tris-HCl (pH 8.0), 1 mM EDTA, 5 mM DTT, 0.05% Triton).
- the homogenate was filtered through two layers of Miracloth and was left to settle at 4°C.
- the supernatant was discarded and the starch enriched pellet was resuspended in 300 µl of thrombin digestion buffer (20 mM Tris-HCl, pH 8.4, 150 mM NaCl, 2.5 mM CaCl₂). 150 µl of this suspension were subjected to digestion with 0.16 U of thrombin for 12 h at 22°C. The remaining 150 µl of the suspension were used as negative control.
- once the digestion with thrombin ended, the sample was centrifuged at 10,000 g for 10 minutes. The supernatant was subjected to SDS-PAGE acrylamide gel electrophoresis and an immunodetection of GFP by means of immunoblotting, making use of a primary anti-GFP antibody.

### 2.2. Identifying the GFP protein

GFP can be identified by any of the following manners: (a) by means of immunoblots making use of specific anti-GFP antibodies and (b) by means of detecting the fluorescence emitted by the GFP protein.

By immunoblotting: The GFP was detected by making use of a specific anti-GFP antibody according to the immunoblotting methodology described in (Towbin *et al.* 1979). The antigen-antibody complex was detected by means of incubation with a rabbit secondary anti-IgG antibody conjugated with alkaline phosphatase.

By analyzing its fluorescence by means of confocal microscopy. Potato and Arabidopsis Plants were transformed with the chimeric GBSS-thr-GFP construct obtained as illustrated in Figure 8. The plants were subjected to observation by means of confocal microscopy to identify the sub-cellular fluorescence localization of GFP (Figure 6).

*N. benthamiana* plants were transformed with the chimeric *GBSS-thr-GFP* construct obtained according to the method illustrated in Figure 7 and in Figure 8. The starch granules obtained from these leaves after a pass of homogenization and subsequent centrifugation at low speed were subjected to the digestion with thrombin to release the GFP protein. By means of an additional pass of centrifugation, the soluble GFP protein was separated from the starch granules, a GFP protein enriched supernatant being obtained. To confirm the release of GFP from the starch granules an immunoblotting was performed using a specific anti-GFP antibody. As observed in Figure 9, in the supernatant of the sample treated with thrombin a protein of approximately 29 kDa corresponding to the GFP released from the starch grains is detected. This protein does not appear when the starch grains are not treated with thrombin, which indicates that the release of the protein bound to the GBSS only takes place in the presence of the protease thrombin. In the insoluble starch granules, the anti-GFP antibody recognizes a protein of 96 kDa corresponding to the fusion protein GBSS-thrombin-GFP.

### Example 3: Confirming the reproducibility of the method in potato plants

To test the reproducibility of the method, the method for obtaining transgenic plants of the section 1.3 was again carried out, in this case in potato plants *(Solanum tuberosum)*

Like Examples 1 and 2, the transgenic plants accumulated starch which expressed the GFP (in this case, the recombinant protein of interest to be purified) fused to the *Arabidopsis thaliana* GBSS as protein with the purpose of binding to the starch granule. Thus, as in the previous example, the protein of interest should be anchored to the starch granule, the high density of which allows an easy purification from the rest of cellular components after a single pass of tissue homogenization, centrifugation and solubilization of the recombinant protein of interest after a digestion treatment with endoprotease.

The system, such as outlined in Figure 4, is divided into two main phases: (1) obtaining starch granules after homogenizing the potato tubers, subsequent filtering of the homogenate through a cloth of *"cheesecloth"* type and centrifuging at 1000g for 5 minutes and (2) releasing the GFP after treating the starch with the corresponding endoprotease (in this case thrombin) and subsequent pass of centrifugation. To homogenize the tubers, a buffer which contained 50 mM (pH 8) Tris-ClH, 1 mM EDTA, 5 mM DTT and 0.05% Triton was used in a ratio of 1 ml of buffer per 1 gram of tuber. To digest the starch with thrombin, the latter was resuspended in a buffered solution which contained 20 mM Tris-ClH (pH 8.4), 150 mM NaCl, 2.5 mM CaCl₂ in a ratio of 10 ml of buffer per 1 gram of starch. This buffered solution contained 0.3 units of thrombin per ml.

The time required for obtaining 1 mg of highly purified GFP from 10 Kg of tubers was 12 hours (5 hours for phase 1 and 7 hours for phase 2).

An analysis, to which the microphotograph of Figure 10 corresponds to, of the tuber starch granules was carried out by confocal microscopy. How the GFP remains active in the potato tuber starch granule giving rise to its characteristic green fluorescence can be seen in the same figure, therefore this new method allows producing and purifying proteins properly folded and assembled from starch obtained from storage organs.

The expression system based on the use of the 35S promoter used in the constructions whereby the transgenic plants were obtained allows GFP accumulation levels of approximately 6 mg per kg of fresh tuber weight (approximately 60 mg of GFP per kg of potato tuber starch). By taking into account that 1 kg of fresh potato tuber weight has approximately 6 g of protein, it is estimated that the GFP involves approximately 0.1% of the total protein of the transgenic tubers.

The purity of the protein was tested by means of electrophoresis once the step of digestion with the endoprotease and the subsequent centrifugation were carried out. The results can be observed in Figure 11, where the results of the following are depicted: SDS- polyacrylamide (PAGE) gel electrophoresis and the subsequent staining with Coomasie Blue (A) or analysis by Western Blot (B) of extracts obtained from (1) potato tuber expressing GFP bound to GBSS through an amino acid sequence specifically recognized by thrombin, (2) pure starch and (3) protein released after treating the pure starch with thrombin. In (1) 10 µg of protein coming from 1.6 mg of fresh tuber weight was loaded. In (2) 1 µg of purified starch protein was loaded and in (3) 0.15 µg of protein released from the purified starch after the treatment with thrombin was loaded. The immunoblot of panel "B" compared with a standard assay in which known amounts of GFP were loaded shows that the 10 µg of protein loaded in (1) contain approximately 10 ng of GFP (0.1% of the total protein), whereas the 150 ng of protein loaded in (3) have approximately 100 ng of GFP (66% of the protein). In other words, in a single pass (purification of starch and treatment with endoprotease) the GFP (a) has been released from the starch granule and (b) has been purified between 600 and 1000 times.

It is emphasized that in a single pass the new method allow purifying 600-1000 times the GFP, obtaining in a simple and fast manner a minimum of 50-100 ng of highly purified GFP from 1 kg of transgenic potato tuber (0.5-1 mg of highly purified GFP from 1 kg of transgenic potato tuber starch).

In this example, the protease used to release the GFP was biotinylated such that its removal from the final preparation is simple. However, SDS-PAGE and subsequent staining of proteins show that the GFP preparation obtained after the phase of "release" from the starch granule has a small contamination from a protein (seer Figure 11 again). In this case, the additional pass of purification which is contemplated as a possible optional step of the method could be convenient.

### Example 4: Extending to proteins of clinical / therapeutic interest

As has been discussed, one of the main uses of the method of the invention is its application for amplifying and purifying proteins of interest in the pharmaceutical sector, many of which are currently being prepared by recombinant methods in which the amplification is made in microorganisms, especially *Escherichia coli.*

As has been discussed, when the recombinant polypeptide of interest is to be used in the pharmaceutical sector, the same must be integral, without lacking any of the amino acids typical of its characteristics sequence and also without having amino acids additional thereto.

To that end, potato lines which will express the *Arabidopsis thaliana* GBSS bound to proteins of therapeutic interest were prepared. The methodology was similar to that described in the previous examples, with the exception that, in this case, the linker fragment connecting the GBSS with the polypeptide of interest did not contain the thrombin recognition sequence, but that of the enterokinase (Asp-Asp-Asp-Asp-Lys), the Lys being bound directly to the first amino acid of the polypeptide of interest in the fusion protein. Thus, since the enterokinase cleaves right after the Lys, the polypeptides of interest can be obtained intact without additional amino acids.

The polypeptides of pharmaceutical interest chosen were the following:
- Palifermin: is a 140 residue N-truncated recombinant form of the human keratinocyte growth factor, KGF (Accession No. UniProt: P21781; GenBank Accession No.: M60828). It is currently produced in *Escherichia coli.* Its specific sequence can be looked up in: http://www.drugbank.ca/drugs/BTD00042. It is a drug used to boost the development of the mucous membrane of the gastrointestinal tracks of cancer patients treated with chemotherapy.
- Mecasermin: human IGF-I (growth factor similar to insulin 1) obtained by means of recombinant growth techniques in *Escherichia coli.* The 70 amino acid sequence is the same as the human IGF-I. The specific sequence can be looked up in: http://www.drugbank.ca/drugs/DB01277 (GenBank Accession No. for the cDNA: X57025).
- Albumin: produced in yeasts from the coding sequence of the mature human protein (in humans, it is synthesized in the form of precursor). It is preferably used in the form of recombinant to prevent contaminations with hepatitis and HIV upon extracting it from serum. It is purified in columns (mature sequence: http://www.drugbank.ca/drugs/DB00062; GenBank Accession No. for the complete CDS: M12523).

With the above data with respect to the sequence of therapeutic interest and its coding sequence, a method similar to that described in Example 1 was carried out, although a plasmid similar to pGWB2-GBSS gtw which also contained the coding sequence of the *Arabidopsis thaliana* GBSS under the control of the 35S promoter is being used as the intermediate plasmid pGBSS gtw. The application of the method allowed generating transgenic potato plants in which a fusion protein of the GBSS with the coding sequence of the corresponding peptide of the interest was expressed. The process for obtaining the necessary plasmids to that end is outlined in Figures 12, 13 and 14, where the structure of the intermediate plasmid pGBSS gtw can also be seen.

The working of the methodology was again carried out with a similar method in which the protein of interest was again the GFP. The process for obtaining the necessary plasmid is outlined in Figure 15. The system, such as outlined in Figure 4, is divided into two main phases: (1) obtaining starch granules after homogenizing the potato tubers and subsequent filtering of the homogenate through a cloth of *"cheesecloth"* type and centrifuging at 1000g for 5 minutes and (2) releasing the GFP after treating the starch with the corresponding endoprotease (in this case enterokinase) and subsequent pass of centrifugation. To homogenize the tubers, a buffer which contained 50 mM (pH 8) Tris-ClH, 1 mM EDTA, 5 mM DTT and 0.05% Triton was used in a ratio of 1 ml of buffer per 1 gram tuber. To digest the starch with enterokinase, the latter was resuspended in a buffered solution in a ratio of 10 ml of buffer per 1 gram of starch which contained 0.3 units of enterokinase per ml. Subsequently it was tested that the GFP released after the treatment with the enterokinase reached the end of process integral from its amino acid sequence point of view (it did not lack amino acids or have excessive amino acids in the amino-terminal part). To that end, the amino terminal end of the purified GFP was sequenced confirming that it corresponded exactly with the sequence of the amino end of the GFP (amino acids 632 to 661 of SEQ NO:2).

The method for the isolation, release of the polypeptides of interest with enterokinase and purity verification are similar to those of Examples 2 and 3 demonstrated above wherein the peptides of interest were obtained in the desired form, with its sequence integral and without vestiges of the amino acid sequence recognized by the endoprotease.

### Literature

Bahaji A., Ovecka M., Barany I., Risueño M.C., Muñoz F.J., Baroja-Fernández E., Montero M., Li J., Hidalgo M., Sesma M.T., Ezquer I., Testillano P.s., Pozueta-Romero J. (2011a) dual targeting to mitochondria and plastids of AtBT1 and ZmBT1, two members of the mitochondrial carrier family. Plant Cell Physiol. 52: 597-609.
Bahaji, A., Li, J., Ezquer, I., Ovecka, M., Muñoz, F.J., Baroja-Fernández,E., Romero, J.M., Montero, M., Hidalgo, M., Sesma, M.T., Pozueta-Romero, J. (2011b) Arabidopsis thaliana mutants lacking ADP-glucose pyrophosphorylase can accumulate high levels of starch and ADP-glucose: further evidences for the occurrence of important sources, other than ADP-glucose pyrophosphorylase, of ADP-glucose linked to leaf starch biosynthesis. Plant Cell Physiol. 52, 1162-1176
Bhojwani S, Razdan MK (1996). Plant tissue culture: theory and practice, Revised edition Elsevier, vii+767 pp
Borisjuk N.V., Borisjuk L.G., Logendra S., Petersen F., Gleba Y. and Raskin I. (1999) Production of recombinant proteins in plant root exudates. Nat. Biotechnol. 17: 466-469.
Clough S.J. and Bent A.F. (1998) Floral dip: a simplified method for Agrobacterium-mediated transformation of Arabidopsis thaliana. Plant J. 16: 735-43.
Enami K, Ozawa T, Motohashi N, Nakamura M, Tanaka K, Hanaoka M. (2011) Plastid-to-nucleus retrograde signals are essential for expression of nuclear starch biosynthesis genes during amyloplast differentiation in tobacco BY-2 cultured cells. Plant Physiology Preview. Published on July 19, 2011, as DOI: 10.1104/pp.111.178897
Fischer R., Stoger E., Schillberg S., Christou P. and Twyman R.M. (2004) Plant based production of biopharmaceuticals. Curr. Opin. Plant Biol. 7: 152-158.
Georgiev M, Weber J, Maciuk A. (2009) Bioprocessing of plant cell cultures for mass production of targeted compounds, Applied Microbiology and Biotechnology, 83:809-823
Giddings G. (2001) Transgenic plants as protein factories. Curr. Opin. Biotechnol. 12: 450-454.
Janecek S and Sevcik J (1999) The evolution of starch binding domain. FEBS Letter 456: 119-125.
Jarvis P, Soll J. 2002. Toc, tic, and chloroplast protein import. Biochim Biophys Acta 1590:177-189.
Joensuu J.J., Conley A.J., Lienemann M., Brandle J.E., Linder M.B. and Menassa R. (2010) Hydrophobin fusions for high-level transient protein expression and purification in Nicotiana benthamiana. Plant Physiol. 152: 622-633.
Houlné, G., Schantz, M.L., Meyer, B., Pozueta-Romero, J. and Schantz, R. (1994) A chromoplast-specific protein in Capsicum annuum: characterization and expression of the corresponding gene. Curr. Genet. 26: 524-527
Kitajima, A., Asatsuma, S., Okada, H., Hamada, Y., Kaneko, K., Nanjo, Y., Kawagoe, Y., Toyooka, K., Matsuoka, K., Takeuchi, M., Nakano, A. and Mitsui, T. (2009) The rice alpha-amylase glycoprotein is targeted from the Golgi apparatus through the secretory pathway to the plastids. Plant Cell 21: 2844-2858
Ma J.K.C., Drake P. and Christou P. (2003) The production of recombinant pharmaceutical proteins in plants. Nature Rev. Genet. 4: 794-805.
Markley N.A., Nykiforuk C.L., Boothe J.G. and Moloney M.M. (2006) Producing proteins using transgenic oil body-oleosin technology. BioPharm International 19: 34-57.
Nagata T, Hasezawa, S; Inzé, D (2004) Biotechnology in Agriculture and Forestry 53: Tobacco BY-2 cells. Springer Verlag
Nanjo, Y., Oka, H., Ikarashi, N., Kanedo, K., Kitajima, A., Mitsui, T., Muñoz, F.J., Rodríguez-López, M., Baroja-Fernández, E., Pozueta-Romero, J. (2006) Rice plastidial N-glycosylated nucleotide pyrophosphatase/phosphodiesterase is transported from the ER-Golgi to the chloroplast through the secretory pathway. Plant Cell 18, 2582-2592
Rocha-Sosa M., Sonnewald U., Frommer W., Stratmann M., Schell J. and Willmitzer L. (1989) Both developmental and metabolic signals activate the promoter of a class I patatine gene. EMBO J. 8, 23-29.
Schillberg S., Zimmermann S., Findlay K. and Fischer R. (2000) Plasma membrane display of anti-viral single chain Fv fragments confers resistance to tobacco mosaic virus. Mol. Breeding 6 :317-326.
Singh SK, Seema Srivastava. (2009). Plant Tissue Culture. Campus Book International.
Towbin H., Staehelin T. and Gordon J. (1979) Electrophoretic transfer of proteins from polyacrylamide gels to nitrocellulose sheets: procedures and some applications. Proc. Natl. Acad. Sci. USA 76: 4350-4354.
Twyman R.M., Schillberg S. and Fischer R. (2005) Transgenic plants in the biopharmaceutical market. Expert Opin. Emerg. Drugs 10: 185-218.
Twyman R.M., Stoger E., Schillberg S., Christou P. and Fischer R. (2003) Molecular farming in plants: host systems and expression technology. Trends Biotechnol. 21: 570-578.
Villarejo, A., Burén, S., Larsson, S, Déjardin, A., Monné, M., Rudhe, C., Karlsson, J., Jansson, S., Lerouge, P., Rolland, N., von Heijne, G., Grebe, M., Bako, L., Samuelsson, G. (2005) Evidence for a protein transported through the secretory pathway en route to the higher plant chloroplast. Nat. Cell Biol. 7, 1124-1131
Voinnet O., Pinto Y.M. and Baulcombe D.C. (1999) Suppression of gene silencing: A general strategy used by diverse DNA and RNA viruses of plants. Proc. Natl. Acad. Sci. USA 96: 14147-14152
Ward WW and Swiatek Gavin. (2009) Protein Purification. Current Analytical Chemistry 5:1-21.

## Claims

1. A method for obtaining purified recombinant proteins from a transgenic plant or transgenic cell culture comprising the following steps:
a) homogenizing the cell culture or a starch-accumulating plant tissue to which is associated a fusion protein **characterized in that** it comprises:
i) in the carboxyl end region, the polypeptide corresponding to the recombinant protein to be purified,
ii) in the amino end region, a starch binding domain,
iii) a linker fragment containing the protease cleavage site, linker fragment which is located between the amino end region and the carboxyl end region;
b) centrifuging and selecting the most dense fraction containing the starch granule proteins;
c) treating the fraction selected in step b) with the protease which recognized the cleavage site comprised in the linker fragment;
d) centrifuging and selecting the supernatant;
e) optionally, purifying the recombinant protein contained in the supernatant in high grade.

2. The method according to claim 1, comprising the prior steps of:
a) i) obtaining a transgenic plant by means of transforming a wild-type plant with a transformation vector comprising a plant expression promoter operatively bound to a coding sequence of the fusion protein comprising, in 5'-3' direction, a peptide directing the transition of cytosol to plastids, a starch binding domain, a protease cleavage site, and the polypeptide corresponding to the recombinant protein to be purified;
a) ii) culturing the transgenic plant obtained in the previous step in conditions suitable for its growth.

3. The method according to claim 1 or 2, in which the starch binding domain is a bacterial amylase binding domain.

4. The method according to claim 3, in which the starch bacterial amylase binding domain is selected from the group consisting of SEQ ID NO:3, SEQ ID NO:4, SEQ ID NO:5 and SEQ ID NO:6.

5. The method according to claim 3 or 4, in which the expression vector whereby the transgenic plant is prepared comprises the coding part corresponding to a suitable transit peptide between the promoter and the starch binding domain.

6. The method according to claim 5, in which the transit peptide is the one represented by SEQ ID NO:8.

7. The method according to claim 1 or 2, in which the starch binding domain belongs to a vegetable protein.

8. The method according to claim 7, in which the fusion protein comprises the complete polypeptide sequence of the vegetable protein which is bound to the starch and which is synthesized in the cytosol in the form of a precursor comprising the complete amino acid sequence of the precursor form of said vegetable protein.

9. The method according to claim 8, in which the expression vector whereby the transgenic plant is prepared comprises the complete natural coding sequence of the starch binding vegetable protein, included the part corresponding to the plastid transit peptide.

10. The method according to claim 9, in which the vegetable protein comprising a starch binding domain can be selected from the group consisting of: starch granule-bound starch synthase (GBSS), glucan-dikinase (R1), one of the starch-branching enzyme from the group consisting of BEI, BEIIa, BEIIb, one of the soluble starch synthases from the group consisting of SSI, SSII, SSIII, and the plastidial starch phosphorylase.

11. The method according to claim 10, in which the coding sequence of the vegetable protein has at least 60% homology with the natural coding sequence of an enzyme selected from the group consisting of: starch granule-bound starch synthase (GBSS), glucan-dikinase (R1), one of the starch-branching enzyme from the group consisting of BEI, BEIIa, BEIIb, one of the soluble starch synthases from the group consisting of SSI, SSII, SSIII, and the plastidial starch phosphorylase with the condition that the enzyme synthesized from said coding sequence has a starch binding domain and in that it has a signaling sequence capable of acting as transit peptide directing the initially synthesized precursor to plastid.

12. The method according to claim 11, in which the coding sequence of the vegetable protein is the natural coding sequence of an enzyme selected from the group consisting of GBSS, R1, BEI, BEIIa, BEIIb, SSI, SSII, SSIII, and plastidial starch phosphorylase.

13. The method according to claim 12, in which the vegetable protein is the GBSS.

14. The method according to any one of the preceding claims, in which the protease used in step c) performs the cleaving after the last amino acid of the recognition sequence thereof.

15. The method according to claim 14, in which the protease is the enterokinase, the linker fragment has at the carboxyl end the enterokinase recognition sequence, Asp-Asp-Asp-Asp-Lys and the amino end of the polypeptide of interest is directly bound to the enterokinase recognition sequence before the protease acts.

16. The method according to claim 15, in which the polypeptide of interest is selected from the group consisting of: palifermin, mecasermin and albumin.

17. The method according to claim 16, in which the vegetable protein having a starch binding domain is the GBSS of *Arabidopsis thaliana.*

18. The method according to any one of the preceding claims, in which the protease used in step c) is thrombin.

19. The method according to any one of the preceding claims, in which the promoter directing the fusion protein expression is the 35S promoter of the cauliflower mosaic virus.

20. The method according to claim 13, in which the vegetable protein having a starch binding domain is the GBSS of *Arabidopsis thaliana,* the linker fragment contains a thrombin cleavage site , the polypeptide of interest bound to the linker fragment is the green fluorescent protein (GFP), the promoter directing the fusion protein expression is that of the RNA 35S, the coding sequence of the fusion protein is the sequence represented by SEQ ID NO:1 and the fusion protein synthesized is represented by SEQ ID NO:2.

21. The method according to any one of claims 2 to 20, in which the transgenic plant is obtained by means of a vector comprising, in 5'-3' direction, a promoter allowing the expression in plants to which there is operatively bound the coding sequence of the fusion protein comprising, in the amino to carboxyl direction, a cytosol-plastid transit peptide, a starch binding domain, a linker fragment containing the protease cleavage site and the polypeptide corresponding to the recombinant protein to be purified.

22. The method according to any one of claims 1 to 21, in which the optional step e) is carried out by a technique selected from the group consisting of size separation, immunoadsorption, ion exchange or fluorescence detection.

23. The method according to any one of claims 1 to 22, in which the starch granules obtained in step b) are subjected to enzymatic digestion or physical rupturing treatments breaking up the starch granules, prior to the application of step c) of applying the protease.

24. The method according to any one of claims 1 to 23, in which the species from which the transgenic plant is obtained is selected from the group consisting of rice, barley, wheat and corn, potato, tobacco, tomato or *Arabidopsis thaliana* plants.

25. The method according to any one of claims 1 to 24, in which the starch-accumulating tissue on which step a) of the method is applied, is selected from the group consisting of tubers, leaves, fruits and seeds.

26. An expression vector for transforming plants for use in the method of any one of claims 1 to 25, comprising, in 5'-3' direction, a promoter allowing the expression in plants, to which there is operatively bound the coding sequence of the fusion protein comprising, in the amino to carboxyl direction, a cytosol-plastid transit peptide, a starch binding domain, a linker fragment containing the protease cleavage site and the polypeptide corresponding to the recombinant protein to be purified.

27. the vector according to claim 26 is a plasmid.

28. The vector according to claim 27, in which the promoter to which is operatively bound the coding sequence of the fusion protein is the 35S promoter.

29. The vector according to any one of claims 27 or 28, in which the cytosol-plastid transit peptide sequence and the starch binding domain are comprised within the natural coding sequence of a vegetable protein which is bound to the starch.

30. The vector according to claim 29, in which the vegetable protein which is bound to the starch is the GBSS.

31. The vector according to any one of claims 27 to 30, in which the linker fragment comprises the coding sequence of thrombin or enterokinase recognition site.

32. The vector according to any one of claims 27 to 31, comprising, bound to the carboxyl end of the linker fragment, the coding sequence of a polypeptide selected from the group consisting of GFP, palifermin, mecasermin, human insulin precursor peptide and mature human albumin.

33. The vector according to any one of claims 27 to 32, in which the coding sequence of the fusion protein is flanked by attB: attB1 and attB2 sequences.

34. A host organism for use in the method of any one of claims 2 to 25 transformed with a vector of any one of claims 26 to 33.

35. The host organism according to claim 34 is *Agrobacterium tumefaciens.*

36. A transformed vegetable cell with the host organism of claim 34 or 35.

37. A transgenic plant, its seeds or propagation material obtained from cells of claim 36.

38. The transgenic plant, its seeds or propagation material, **characterized in that** its genome integrates, in 5'-3' direction, a promoter allowing the expression in plants, to which is operatively bound the coding sequence of the fusion protein comprising, in the amino to carboxyl direction, a cytosol-plastid transit peptide, a starch binding domain, a linker fragment containing the protease cleavage site and the polypeptide sequence of a protein not naturally expressed in the species to which the transgenic plant belongs.

39. An intermediate vector for generating a vector of any one of claims 26 to 33, **characterized in that** it comprises, in 5'-3' direction, a promoter allowing the expression in plants, to which is operatively bound the coding sequence of a vegetable protein which is bound to starch, coding sequence which is followed by a fragment flanked by attR1 and attR2 sequences.

40. The vector according to claim 39 is a plasmid.

41. The vector according to claim 40, in which the encoded vegetable protein is the GBSS.

42. The vector according to claim 41, in which the GBSS is that of *Arabidopsis thaliana* or that of *Solanum tuberosum.*

43. A bacterial host transformed with a vector of any one of claims 39 to 42.

44. The host according to claim 43 belongs to the *Escherichia coli* bacterial species.
